(19) Europäisches Patentamt / European Patent Office / Office européen des brevets

(11) **EP 4 487 863 A1**

(12) **EUROPEAN PATENT APPLICATION**

(43) Date of publication:
**08.01.2025 Bulletin 2025/02**

(21) Application number: **23184221.2**

(22) Date of filing: **07.07.2023**

(51) International Patent Classification (IPC):
**A61K 38/08** (2019.01) **A61K 31/728** (2006.01)
**A61K 38/10** (2006.01) **A61L 27/52** (2006.01)
**A61P 19/02** (2006.01)

(52) Cooperative Patent Classification (CPC):
(C-Sets available)
**A61K 38/08; A61K 31/728; A61K 38/10;**
**A61L 27/20; A61L 27/52; A61L 27/54; A61P 19/02;**
A61K 2300/00; A61K 2300/252; A61L 2400/06;
A61L 2430/24 (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **NuPep AS**
**1176 Oslo (NO)**

(72) Inventors:
• **ØVREBØ, Øystein**
 **1176 Oslo (NO)**

• **ZAIDI, Rija Fatima**
 **0671 Oslo (NO)**
• **PERALE, Giuseppe**
 **6925 Collina d'Oro (CH)**

(74) Representative: **Aera A/S**
 **Niels Hemmingsens Gade 10, 5th Floor**
 **1153 Copenhagen K (DK)**

Remarks:
The complete document including Reference Table(s) and the Sequence Listing(s) can be downloaded from the EPO website

(54) **CONSENSUS PEPTIDES IN HYA FOR USE IN TREATING DEGENERATED JOINTS**

(57) Pharmaceutical formulations are described and their uses in medicine comprising artificial peptides characterized by a proline sequence prevalent in key matricellular proteins that partake in wound healing as well as in bone and cartilage formation and connective tissue maintenance in all vertebrates.

The current invention relates to a pharmaceutical formulation in the form of a gel comprising a. an artificial peptide comprising the amino acid sequence of Pro-X-X-Pro-Y-Y-Y-Pro-X-X-Pro-Y-Y-Pro-X-X-Pro-X- Pro-Y-Y-Y-Y-Y-Pro-Y-Y-Y-Y-Y-Pro-X-X-Pro-X-Pro-Y-Y-Pro-Y-Y-Pro-Y-Pro-X-X-Pro-Y-Pro-Y-Y-Pro-X-X- Pro-Y-Y-Pro-X-X-Pro-Y-Y-Pro-X-X-Pro-Y-Pro-Pro-X-Pro-Pro-X-X-X-X-X-X-X-X-Pro-X-X-Pro-X-X-X-X (SEQ ID NO 1) and/or an artificial peptide comprising or consisting of the amino acid sequence of Pro-X-X-Pro-Y-Y-Pro-X-X-Pro-Y-Y-Pro-X-X-Pro-Y-Y- Pro-Y-Pro-Pro-X-Pro-Pro (SEQ ID NO 2), wherein i) Pro is proline; ii) X is an amino acid selected from the group consisting of Ala, Ile, Leu, Met, Phe, Trp and Val; iii) Y is an amino acid selected from the group consisting of Asn, Cys, Gln, Ser, Thr and Tyr, b. cross-linked Hyaluronicfibres (HA-XL), and/or c. non-crosslinked Hyaluronic fibres (HA), for use in treating inflamed, degenerated and/or damaged joints.

EP 4 487 863 A1

(52) Cooperative Patent Classification (CPC): (Cont.)

C-Sets
**A61L 27/20, C08L 5/08**

**Description**

TECHNICAL FIELD

The present invention relates to the field of tissue healing and/or regeneration for treating inflamed, degenerated and/or damaged joints.

Pharmaceutical formulations are described comprising artificial peptides characterized by a proline sequence prevalent in key matricellular proteins that partake in wound healing as well as in bone and cartilage formation and connective tissue maintenance in all vertebrates and their uses in medicine. The artificial peptides are herein formulated in hydrogels, comprising hyaluronic acids or crosslinked hyaluronic acids and/or combinations thereof for minimally invasive administration and/or controlled release, for use in the treatment of degenerative and/or inflammatory tissue conditions, damages, diseases and/or disorders in joints and/or ligaments, such as e.g., arthritis, rheumatoid arthritis and/or osteoarthritis.

BACKGROUND

Arthritis means inflammation or swelling of one or more joints. It describes more than 100 conditions that affect the joints, tissues around the joint, and other connective tissues. Specific symptoms vary depending on the type of arthritis, but usually include joint pain and stiffness.

Osteoarthritis (OA) is the most common form of arthritis. Some people call it degenerative joint disease or "wear and tear" arthritis as it may arise from wear and tear on the joints, as well as genetic predisposition, joint injury or trauma. It occurs most frequently in the hands, hips, shoulders, elbows and knees, but is also often seen in fingers, wrists and toes. With OA, the cartilage within a joint begins to break down and the underlying bone begins to change.

Rheumatoid arthritis, or RA, is an autoimmune and inflammatory disease, wherein the immune system attacks healthy cells in the body by mistake, causing inflammation in the affected parts of the body. RA mainly attacks the joints, usually many joints at once.

In general, joints are the areas where two or more bones meet. Most joints are mobile, allowing the bones to move. Joints consist of cartilage, synovial membrane, ligaments, tendons, bursas and synovial fluid.

Cartilage is composed of specialized cells called chondrocytes embedded within a matrix of collagen fibres and proteoglycans, and unlike bone, cartilage lacks blood vessels and nerves, which limits its ability to heal and regenerate.

Cartilage covers the articulating surfaces of bones in joints, acting as a smooth and lubricated surface, where it reduces friction and absorbs shock during movement protecting bones from wear and tear. While cartilage has limited regenerative capacity, it can undergo repair to some extent. This process is slow and often results in the formation of fibrous scar tissue rather than the complete restoration of healthy cartilage.

Degenerative and/or inflammatory tissue disorders, diseases and damages can affect all types of tissue in the joints. They can be caused by trauma, repeated trauma, infection, wear and tear, systemic disorders and autoimmune diseases.

Disorders such as osteoporosis, rheumatoid arthritis, and osteoarthritis to some extend all lead to inflammation of the joints. Osteoarthritis occurs frequently in the aging population and varies in severity from mild inconveniences to severe disabling and incapacitating pain and joint stiffness, overall, severely affecting the quality of life for the patient.

Biomaterial scientists design in particular organic bone substitutes based on the biochemical properties of the mimicked tissue to achieve near native functionality. Several non-collagenous proteins in bone are known as intrinsically disordered proteins (IDPs), as they lack detectible ordered domains and a fixed 3D structure under physiological conditions.

EP2118136 discloses artificial IDPs peptides with improved properties for induction and/or stimulation of mineralization, in vivo and in vitro. Such peptides are provided which are easy to synthesize and methods of using the peptides for the induction and/or stimulation of mineral precipitation and/or biomineralization.

The characterizing sequence of amino acids of the artificial peptides disclosed in EP2118136 is a proline rich sequence prevalent in key matricellular proteins that partake in wound healing as well as in bone and cartilage formation and connective tissue maintenance in all vertebrates. This core sequence of prolines is highly conserved in vertebrates, is intrinsically disordered and does not induce any immunogenic response in humans.

Joint tissue healing remains challenging e.g., due to the limited blood-flow in some tissue, in particular cartilage. Thus, there is today still a largely unmet need for potent solutions for joint tissue healing and regeneration. In addition, because of the susceptibility of the tissue in the joint to develop post-surgery and/or post-treatment complications and disorders, it would be preferable to find ways for minimally invasive administrations for the delivery of such advanced peptide-technology.

Hydrogels are an extensively investigated class of biomaterials, and an increasing number of products have reached the clinic. Hydrogels represent a group of biomaterials consisting of water- swollen polymer or colloidal networks.

Hydrogels are viscoelastic materials that have attracted attention in regenerative medicine due to their ability to structurally mimic the extracellular matrix (ECM), thereby creating a conducive environment for cell proliferation and tissue regeneration. The viscoelastic properties of hydrogels allow them to function as stem cell carriers or scaffolds for controlled drug release. There is a need for widely different applications for diverse tissues with different loading modes and levels and different clinical requirements of the material. Consequently, a one-fit-all hydrogel is an unlikely strategy.

The current invention addresses both the need for advanced peptide-technology to promote healing and regeneration of tissue in and surrounding the joint, as well as the need for novel means of minimally invasive administration of such active substances to and/or into the tissue or the surrounding tissue affected, in particular the cartilage.

SUMMARY

The current inventors have shown that the artificial IDPs peptides disclosed in EP2118136 are surprisingly not only effective for stimulating regrowth of mineralized tissues, but can also reduce inflammation in joint tissue, and modulate acute and chronic inflammation, to heal and/or regenerate e.g., cartilage tissue, tendons and/or ligaments and to be able to reduce pain and swelling when applied locally. The inventors found that the pharmaceutical formulation disclosed herein was exceptionally suited to extend the release of the peptides described in EP2118136. This is particularly suitable for healing and/or regeneration of inflamed joint tissue, resulting from chronic and/or acute joint inflammation from chronic or acute damage, diseases, disorders and/or conditions, such as e.g., arthritis. In addition, as a secondary effect from the reduced inflammation and healing of the degenerative cartilage, tendons and/or ligaments, the pharmaceutical formulation disclosed herein also reduced inflammation in the bone tissue adjacent to the treated joint.

To address the need for treating inflamed and/or degenerative joint tissue in conditions diseases and disorders such as e.g., arthritis, the current inventors have developed a novel hydrogel formulation for the artificial IDPs peptides that enables minimally invasive administration of the same to joint tissue, which at the same time enables extended release of the peptide from the formulation, overall aiding the healing and/or regeneration of the damaged and/or inflamed tissue in and/or adjacent to the joint.

The current invention thus relates to a pharmaceutical formulation in the form of a gel, such as a hydrogel, comprising a. an artificial peptide characterized by a proline sequence prevalent in key matricellular proteins that partake in wound healing as well as in bone and cartilage formation and connective tissue maintenance in the majority of vertebrates, b. cross-linked Hyaluronic fibres (HA-XL)and/or c. non-crosslinked Hyaluronic fibres (HA), as well as to its uses in tissue healing and/or regeneration in inflamed, degenerated and/or damaged joints, in particular for use in the treatment of an inflamed, degenerated and/or damaged joint.

The current invention in particular relates to a pharmaceutical formulation in the form of a gel, such as a hydrogel, comprising or consisting of a. an artificial peptide comprising the amino acid sequence of Pro-X-X-Pro-Y-Y-Y-Pro-X-X-Pro-Y-Y-Pro-X-X-Pro-X- Pro-Y-Y-Y-Y-Y-Pro-Y-Y-Y-Y-Y-Pro-X-X-Pro-X-Pro-Y-Y-Y-Pro-Y-Y-Pro-Y-Pro-X-X-Pro-Y-Pro-Y-Y-Pro-X-X- Pro-Y-Y-Pro-X-X-Pro-Y-Y-Pro-X-X-Pro-Y-Pro-Pro-X-Pro-Pro-X-X-X-X-X-X-X-Pro-X-X-Pro-X-X-X (SEQ ID NO 1), wherein i) Pro is proline; ii) X is an amino acid selected from the group consisting of Ala, Ile, Leu, Met, Phe, Trp and Val; iii) Y is an amino acid selected from the group consisting of Asn, Cys, Gln, Ser, Thr and Tyr, b. 1-40 mg/mL, such as 1.0, 2.5, 4, 10, 20, 25, or 40 mg/mL, or such as 1-30 mg/mL cross-linked Hyaluronic fibres (HA-XL), and/or c.1-40, such as 1.0, 2.5, 4, 10, 20, 25 or 40 mg/mL or such as 1-30 mg/mL non-crosslinked Hyaluronic fibres (HA), for use in treatment of an inflamed, degenerated and/or damaged joint.

Typically, a pharmaceutical formulation for use according to the current invention comprises or consists of a. 0.1-250 μg/mL, such as 0.1, 1.0, 5.0, 10, 50, 100, 200 or 250 μg/mL of an artificial peptide comprising the amino acid sequence of Pro-X-X-Pro-Y-Y-Y-Pro-X-X-Pro-Y-Y-Pro-X-X-Pro-X- Pro-Y-Y-Y-Y-Y-Pro-Y-Y-Y-Y-Y-Pro-X-X-Pro-X-Pro-Y-Y-Y-Pro-Y-Y-Pro-Y-Pro-X-X-Pro-Y-Pro-Y-Y-Pro-X-X-   Pro-Y-Y-Pro-X-X-Pro-Y-Y-Pro-X-X-Pro-Y-Pro-Pro-X-Pro-Pro-X-X-X-X-X-X-X-Pro-X-X-Pro-X-X-X (SEQ ID NO 1), b.1-40 mg/mL, such as 1.0, 2.5, 4, 10, 20, 25 or 40 mg/mL cross-linked Hyaluronic fibres (HA-XL), and/or c.1-40, such as 1.0, 2.5, 4, 10, 20, 25 or 40 mg/mL mg/mL non-crosslinked Hyaluronic fibres (HA).

Alternatively, or in addition, a pharmaceutical formulation for use according to the current invention comprises or consists of an artificial peptide which comprises the amino acid sequence of Pro-X-X-Pro-Y-Y-Pro-X-X-Pro-Y-Y-Pro-X-X-Pro-Y-Y- Pro-Y-Pro-Pro-X-Pro-Pro (SEQ ID NO 2).

Typically, a pharmaceutical formulation for use according to the current invention comprises a. 0.1-250 μg/mL, such as 0.1, 1.0, 5.0, 10, 50, 100, 200 or 250 μg/mL of an artificial peptide which comprises or consist of the amino acid sequence of Pro-X-X-Pro-Y-Y-Pro-X-X-Pro-Y-Y-Pro-X-X-Pro-Y-Y- Pro-Y-Pro-Pro-X-Pro-Pro (SEQ ID NO 2), b.1-40 mg/mL, such as 1.0, 2.5, 4, 10, 20, 25 or 40 mg/mL cross-linked Hyaluronic fibres (HA-XL), and/or c.1-40, such as 1.0, 2.5, 4, 10, 20, 25 or 40 mg/mL mg/mL non-crosslinked Hyaluronic fibres (HA).

In embodiments, a pharmaceutical formulation for use according to the current invention comprises one or more artificial peptide(s) selected from the group consisting of artificial peptides comprising the amino acid sequence of SEQ ID NO 4 (P2) (peptide 2) and SEQ ID NO 5 (P6), or wherein the artificial peptide is at least 90% identical to an artificial peptide selected from the group consisting of the amino acid sequences of SEQ ID NO 4 and SEQ ID NO 5.

A pharmaceutical formulation for use according to the current invention can comprise cross-linked Hyaluronic fibres (HA-XL) which comprise or consist of 1,4-butanediol diglycidyl ether cross-linked Hyaluronic fibres (HA-BDDE) and/or diglycidyl ether cross-linked Hyaluronic fibres (HA-BDDE or HA-PEGDE).

In a currently preferred embodiment, a pharmaceutical formulation for use according to the current invention comprises a.50 μg/mL of an artificial peptide with the amino acid sequence of SEQ ID NO 4 (P2) and/or SEQ ID NO 5 (P6), b. 20 mg/mL 1,4-butanediol diglycidyl ether cross-linked Hyaluronic fibres (HA-BDDE) and/or diglycidyl ether cross-linked Hyaluronic fibres (HA-BDDE or HA-PEGDE), and c. 2.5 mg/mL non-crosslinked Hyaluronic fibres (HA).

In a pharmaceutical formulation for use according to the current invention, the Hyaluronic acid fibres are typically between 0.7-4.0 MDa, such as between 1.0-2.0, between 1.5-1.8, or between 3.0-3.3 MDa. In one embodiment, the Hyaluronic acid fibres are between 3.0-3.3 MDa, such as 1.5 MDa.

A typical pharmaceutical formulation for use according to the current invention is a hydrogel.

A pharmaceutical formulation for use according to the current invention can further comprise, d. one or more buffering agent(s), e. one or more salt(s), and f. water.

In addition, a pharmaceutical formulation for use according to the current invention can further comprise one or more of a component selected from the group consisting of d. Water, e. Sodium Hydroxide, f. Sodium Chloride, g. Disodium Phosphate and h. Sodium Phosphate.

A pharmaceutical formulation for use according to the current invention can further comprise one or more of a component selected from the group consisting of sorbitol, xylitol, NaOH, HCl, phosphate-buffered saline (PBS), acetic acid, citric acid and maleic acid.

A pharmaceutical formulation for use according to the current invention is stable/in gel form at RT for at least 1-2 years and/or in the body for at least 30 days.

In one aspect, a pharmaceutical formulation for use according to the current invention releases the comprised artificial peptide at a controlled rate, such as of 1μg per hour.

In embodiments, a pharmaceutical formulation for use according to the current invention can further comprise another active ingredient, such as, but not limited to, an active ingredient selected from the group consisting of growth factors, plasma rich fibrin/plasma and enamel matrix derivatives.

One aspect relates to the novel pharmaceutical formulation described herein for use in medicine, in particular uses wherein the pharmaceutical formulation induces and/or supports tissue healing and/or regeneration in a joint and/or in tissue adjacent to a joint, such as e.g., by use in viscosupplementation. In embodiments, the novel pharmaceutical formulation reduces and/or suppresses an infection and/or inflammation in a joint and/or in tissue adjacent to a joint. In further embodiments the pharmaceutical formulation is for use in stimulating collagen production in joint tissue and/or promotes oriented collagen formation in joint tissue.

In preferred aspects, the novel pharmaceutical formulation described herein is for use in preventing, ameliorating, prevent progression of and/or treating arthritis.

The novel pharmaceutical formulation according to the current invention can be for use in an anti-inflammatory and/or antimicrobial treatment, for use in treating cartilage regeneration. In embodiments, the pharmaceutical formulation is administered as a minimally invasive administration by injection.

One aspect of the current invention relates to a method for producing a pharmaceutical formulation for use according to the current invention, said method comprising, a. providing an artificial peptide as defined herein, b. providing hyaluronic acid (HA) with a molecular weight of 0.7-4 MDa, such as between 1.0-2.0, between 1.5-1.8, or between 3.0-3.3 MDa, such as 1.5 MDa, wherein said Hyaluronic acid may comprise or consist of BDDE and/or PEGDE crosslinked hyaluronic acid, c. mixing 0.1-250 μg/mL of said artificial peptide and 1-40 mg/mL of said HA and d. optionally, adding a source of fluoride to said mixture, wherein said pharmaceutical formulation has an osmolarity of 50-400 mOsm/L, such as between 100 and 310 mOsm/L, or such as between 125 and 175 mOsm/L, such as about 150 mOsm/L, or such as between 275 and 325 mOsm/L, such as about 300 mOsm/L. Preferably, said Hyaluronic acid comprises or consists of BDDE and/or PEGDE crosslinked hyaluronic acid.

Consequently, the current invention also relates to a pharmaceutical formulation obtained by a method according to the current invention, as well as to its use in medicine, in particular to its use in treating arthritis.

DEFINITIONS AND ABBREVIATIONS

In the present context, an "artificial peptide" refers to a peptide that is a non-natural peptide in the sense that it does not normally occur in nature but is the product of amino acids put together and selected in an order, amount and manner generating peptides suitable for use in the context of the present invention. An "artificial peptide" is still a peptide embraced by the present invention even though it might encompass parts of or a whole peptide which happens to be present in nature. "Artificial" may be used interchangeably with terms such as "synthetic" or "non-natural".

In the present context "Pro" denotes the amino acid proline.

In the present context "X" denotes a hydrophobic amino acid. A hydrophobic amino acid is, in the present context, defined as an amino acid selected from the group consisting of: Ala, Ile, Leu, Met, Phe, Trp and Val.

In the present context "Y" denotes a polar amino acid. A polar ("hydrophilic") amino acid is, in the present context, defined as an amino acid selected from the group consisting of: Asn, Cys, Gln, Ser, Thr and Tyr.

In the present context, common nomenclature is used for denoting amino acids. Therefore, for example, A is Ala (hydrophobic), C is Cys (polar), F is Phe (hydrophobic), H is His, I is Ile (hydrophobic), L is Leu (hydrophobic), M is Met (hydrophobic), N is Asn (polar), Q is Gln (polar), S is Ser (polar), T is Thr (polar), V is Val (hydrophobic), W is Trp (hydrophobic), Y is Tyr (polar).

In the present context "surface" refers to any surface which may be of interest to provide with an artificial peptide of the invention, such as a metal surface, e.g. a titanium, zirconium, tantalum, aluminium, gold, surgical steel or a nickel surface, or an alloy thereof, or a metal oxide surface thereof, or a metal hydroxide or metal hydride surface thereof, or a hydroxyl apatite, aragonite, bioglass, glass, or polyurethane surface. Another example of a surface according to the invention is a biological surface such as a graft surface, a wound surface etc. Additional examples of surfaces comprise mucosal surfaces, skin surfaces and other equivalent surfaces.

In the present context "subject" relate to any vertebrate animal, such as bird, reptiles, mammals, primates and humans.

HA: Hyaluronic acid; Hyaluronic acid (HA) is a natural and linear carbohydrate polymer belonging to the class of non-sulphated glycosaminoglycans. It is composed of beta-1,3-N-acetyl glucosamine and beta-1,4-glucuronic acid repeating disaccharide units with a molecular weight (MW) up to 6 MDa. HA is present in hyaline cartilage, synovial joint fluid, and skin tissue, both dermis and epidermis.

BDDE: 1,4-butanediol diglycidyl ether is a class of crosslinking agents, with a molecular weight which may be designed for the particular use, e.g., a molecular weight (Mw) of about 200 Da.

PEGDE: poly(ethylene glycol) diglycidyl ether, is a class of crosslinking agents with a repetitive poly(ethylene glycol) motif, with a molecular weight which may be designed for the particular use, e.g., a molecular weight (Mw) of about 500 Da.

The term "biocompatible" as used herein refers to causing no clinically relevant tissue irritation, injury, toxic reaction, or immunological reaction to living tissue.

The term "cell" is used herein to refer to the structural and functional unit of living organisms and is the smallest unit of an organism classified as living.

The term "compatible" as used herein means that components of a composition are capable of being combined with each other in a manner such that there is no interaction that would substantially reduce the efficacy of the composition under ordinary use conditions.

The term "component" as used herein refers to a constituent part, element, or ingredient.

The term "condition" as used herein refers to a variety of health states and is meant to include disorders or diseases caused by any underlying mechanism or disorder, injury, and the promotion of healthy tissues and organs.

The term "differentiation" as used herein refers to the process of development with an increase in the level of organization or complexity of a cell or tissue, accompanied with a more specialized function.

The terms "disease" and "disorder" as used herein refer to an impairment of health or a condition of abnormal functioning.

The term "patient" as used herein refers to any mammal. Examples of mammals are humans, farm animals and domestic animals.

The term "peptide" is used herein to refer to two or more amino acids joined by a peptide bond.

It must be noted that as used herein and in the appended claims, the singular forms "a", "an", and "the" include plural references unless the context clearly dictates otherwise.

As used herein, the term "comprising" means the presence of the stated features, integers, steps, or components as referred to in the claims, but that it does not preclude the presence or addition of one or more other features, integers, steps, components or groups thereof. The term "comprising" is intended to include embodiments encompassed by the terms "consisting essentially of' and "consisting of'. Similarly, the term "consisting essentially of' is intended to include embodiments encompassed by the term "consisting of'.

As used herein, the term "about" modifying the quantity of an ingredient or reactant employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture. Whether or not modified by the term "about", the claims include equivalents to the quantities.

In the current context, the abbreviation "u", e.g., as used in "uM" or "ug" or "uL", is used interchangeably with "micro", "mcg" or "$\mu$". E.g., a microgram or microgramme is a unit of mass equal to one millionth ($1\times10^{-6}$) of a gram.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding both of those included limits are also included in the invention.

It is to be understood that the terminology used herein is for the purpose of describing particular embodiments only and is not intended to limit the scope of the present invention which will be limited only by the appended claims.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, exemplary methods and materials have been described. All publications mentioned herein are incorporated to disclose and describe the methods and/or materials in connection with which the publications are cited.

BRIEF DESCRIPTION OF THE DRAWINGS

Figure 1: Schematic representation of the production steps for the manufacturing of a hyaluronic acid gel crosslinked with either BDDE or PEGDE.

Figure 2: A, Rheology shear and frequency sweeps of the HA-BDDE and HA-PEGDE gel. B, Real-time crosslinking monitoring of the two gels over a 5h time period at 40 °C. C FTIR analysis of the two gels. D SEM analysis of the cross-section of the two gels. The crystals observed for the HE-PEGDE are sodium chloride formed during the neutralization step.

Figure 3: $^1$H NMR of leaching medium of A. BDDE-HA or B. PEGDE-HA, before (top panels in A and B) and after 18h dialysis (bottom panels in A and B) in distilled water.

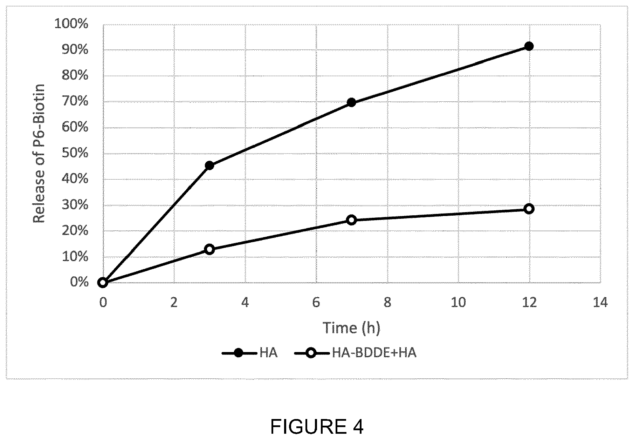

Figure 4: Comparison of release of P6 biotin from crosslinked HA-BDDE and non-crosslinked HA gels.

DETAILED DESCRIPTION

The current invention relates to a pharmaceutical formulation in the form of a gel, such as a hydrogel, comprising a. an artificial peptide characterized by a proline sequence prevalent in key matricellular proteins that partake in wound healing as well as in bone and cartilage formation and connective tissue maintenance in the majority of vertebrates, b. cross-linked Hyaluronic fibres (HA-XL), and/or c. non-crosslinked Hyaluronic fibres (HA). In particular, the current invention relates to a pharmaceutical formulation as described herein for use in ameliorating, treating, prevent progression of and/or preventing arthritis.

In one aspect, the formulation of the current invention comprises one or more artificial peptides loaded into a hydrogel carrier. The one or more artificial peptide(s) comprised in the formulation of the current invention is characterized by comprising or consisting of the amino acid sequence of Pro-X-X-Pro-Y-Y-Y-Pro-X-X-Pro-Y-Y-Pro-X-X-Pro-X- Pro-Y-Y-Y-Y-Y-Pro-Y-Y-Y-Y-Y-Y-Pro-X-X-Pro-X-Pro-Y-Y-Y-Pro-Y-Y-Pro-Y-Pro-X-X-Pro-Y-Pro-Y-Y-Pro-X-X- Pro-Y-Y-Pro-X-X-Pro-Y-Pro-Pro-X-Pro-Pro-X-X-X-X-X-X-X-Pro-X-X-Pro-X-X-X-X (SEQ ID NO 1), wherein i) Pro is proline; ii) X is an amino acid selected from the group consisting of Ala, Ile, Leu, Met, Phe, Trp and Val; iii) Y is an amino acid selected from the group consisting of Asn, Cys, Gln, Ser, Thr and Tyr.

Alternatively, or in addition, the one or more artificial peptide(s) comprised in the formulation of the current invention is characterized by comprising or consisting of the amino acid sequence of Pro-X-X-Pro-Y-Y-Pro-X-X-Pro-Y-Y-Pro-X-X-Pro-Y-Y- Pro-Y-Pro-Pro-X-Pro-Pro (SEQ ID NO 2), wherein i) Pro is proline; ii) X is an amino acid selected from the group consisting of Ala, Ile, Leu, Met, Phe, Trp and Val; and iii) Y is an amino acid selected from the group consisting of Asn, Cys, Gln, Ser, Thr and Tyr.

In one embodiment, the formulation is delivered via a syringe in a minimally invasive manner. In vitro and in vivo trials suggest that the herein described gel-formulation with peptides has anti-inflammatory effect and stimulates tissue formation in tissues in or adjacent to a joint. Additionally, the formulation of the current invention is fully synthetic, which will e.g., allow scalability and price competitiveness. Moreover, the herein disclosed formulation does not require the use of animal tissue. The use of hydrogel carrier of the current invention uses covalently crosslinked hyaluronic acid, thereby overcoming limitations, such as, but not limited to, rapid clearance after administration.

**The artificial peptide**

In one aspect, the current invention relates to the surprising insight that the artificial peptides disclosed in EP2118136 can be used to promote connective tissue cells and to, through them, have a secondary promotional effect on epithelial cell growth and spreading, e.g., to promote local vascularization and to modulate acute and chronic inflammation, to rejuvenates senescent cells (e.g., damaged from irradiation damage) and to be able to reduce pain and swelling when applied locally.

The artificial peptides according to the current invention are biomimetic peptides inspired by the motifs found in amelogenins. Due to intrinsic disorder, they are flexible peptides that dynamically adopt to the local environment. They are thereby able to interact with other structural polymers such as collagen, giving an improved appearance. This is e.g., done by the peptides folding into a temporary extracellular matrix that can e.g., bind to surfaces of membranes. This provides a prophylactic function, which is especially suited to ameliorate, preventing and/or prevent progression of joint diseases such as e.g., arthritis.

The artificial peptides according to the current invention are biomimicking, intrinsically disordered proteins (IDPs).

Under physiological conditions i.e., conditions of the external or internal milieu that may occur in nature for an organism, such as a human being, the peptides are generally intrinsically disordered. The release of peptides from a gel matrix is usually tied to the structure of the peptides/proteins, wherein intrinsically disordered proteins/peptides are generally more easily released from the gel than structured proteins and/or peptides, due to their high degree of conformational flexibility. This feature makes the artificial peptides of the current disclosure highly useful as constituent to be released from a gel, such as a crosslinked gel.

One of the advantages of using synthetic peptides is that it is not always practical to use natural peptides and/or proteins for medical uses. For example, natural proteins are often long, which means that they are difficult to synthesize, both chemically and by cellular expression systems. Typically, artificial peptides are easier to synthesize than full, larger proteins, as peptides often lack the complicated higher order structure that make large proteins difficult to synthesize. Also, a natural protein only contains natural amino acids and may therefore be susceptible to rapid degradation. Also, if purified from a natural environment, such as developing teeth, there is always a risk of contamination of other products which e.g., may cause allergic reactions. In addition, a long natural protein normally has many roles in a living body and may therefore not be optimized for the intended use.

Furthermore, the active motif of the synthetic peptides in the formulation of the current invention can be designed to specifically stimulate healing and/or growth of tissue in and adjacent to a joint.

The artificial peptide(s) comprised in the formulation of the current invention is/are characterized by a proline sequence prevalent in key matricellular proteins that partake in wound healing as well as in bone and cartilage formation and connective tissue maintenance in the majority of vertebrates.

The artificial peptide(s) comprised in the formulation of the current invention is/are characterized by comprising or consisting of the amino acid sequence of Pro-X-X-Pro-Y-Y-Y-Pro-X-X-Pro-Y-Y-Pro-X-X-Pro-X- Pro-Y-Y-Y-Y-Y-Pro-Y-Y-Y-Y-Y-Pro-X-X-Pro-X-Pro-Y-Y-Y-Pro-Y-Y-Pro-Y-Pro-X-X-Pro-Y-Pro-Y-Y-Pro-X-X- Pro-Y-Y-Pro-X-X-Pro-Y-Y-Pro-X-X-Pro-Y-Pro-Pro-X-Pro-Pro-X-X-X-X-X-X-X-X-Pro-X-X-Pro-X-X-X-X (SEQ ID NO 1), wherein i) Pro is proline; ii) X is an amino acid selected from the group consisting of Ala, Ile, Leu, Met, Phe, Trp and Val; iii) Y is an amino acid selected from the group consisting of Asn, Cys, Gln, Ser, Thr and Tyr.

Alternatively, or in addition, the one or more artificial peptide(s) comprised in the formulation of the current invention is characterized by comprising or consisting of the amino acid sequence of Pro-X-X-Pro-Y-Y-Pro-X-X-Pro-Y-Y- Pro-X-X-Pro-Y-Y- Pro-Y-Pro-Pro-X-Pro-Pro (SEQ ID NO 2), wherein i) Pro is proline; ii) X is an amino acid selected from the group consisting of Ala, Ile, Leu, Met, Phe, Trp and Val; and iii) Y is an amino acid selected from the group consisting of Asn, Cys, Gln, Ser, Thr and Tyr.

Furthermore, the peptides according to the invention may comprise between 20 and 120 amino acids, such as, but not limited to, between 20-25, 25-30, 30-35, 35-40, 40-45, 45-50, 50-60, 60-70, 70-80, 80-90, 90-100, or between 100-120 amino acids, such as, but not limited to, 21, 22, 23, 24, 26, 27, 28, 29, 30, 32, 33, 37, 42, 47, 49, 51, 53, 57, 59, 63, 65, 75, 85, 87, 88, 92, 95, 105, or 115 amino acids. In a preferred embodiment, the peptides according to the invention comprises between 20-50 amino acids.

The amino acids in an artificial peptide of the invention may further be modified in terms of chemistry, isometry or in any other way, as long as the sequences of the peptides are intact. Modifications of the amino acids of the artificial peptides of the invention may increase the activity, stability, biocompatibility, or clinical performance of the peptides, or reduce toxicity and adverse reactions to the peptides. Examples of chemical modifications include, but are not limited to, glycosylation and methylation. The amino acids may also be of all different types of stereoisomeric forms, such as D or L forms of amino acids, or S or R isomers. The amino acids in an artificial peptide of the invention may also be replaced by synthetic analogues thereof. The use of synthetic analogues may e.g., result in a peptide that is more stable and less prone to degradation. Examples of unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylaianine*, p-I-phenylatanine*, L-allyl-glycine*, β-alanine*, L-a-amino butyric acid*, L-g-amino butyric acid*, L-a-amino isobutyric acid*, L-e-amino caproic acid#, 7-amino heptanoic acid*, L-methionine sulfone#*, L-norleucine*, L-norvaline*, p-nitro-L-phenylalainine*, L-hydroxyproline#, L-thioproline*, methyl derivatives of phenylalanine (Phe) such as 4-methyl-Phe*, pentamethyl-Phe*, L-Phe (4-amino)#, L-Tyr (methyl)*, L-Phe (4-isopropyl)*, L-Tic (1,2,3,4-tetrahydroisoquinoline-3-carboxyl acid)*, L-diaminopropionic acid # and L-Phe (4-benzyl)*. The notation * is herein utilised to indicate the hydrophobic nature of the derivative whereas # is utilised to indicate the hydrophilic nature of the derivative, #* indicates amphipathic characteristics.

The above identified peptides are artificial (synthetic) peptides comprising a poly-proline consensus sequence, further comprising hydrophobic ("X") and polar amino acids ("Y"). It induces and/or stimulates soft tissue healing in biological systems, and that also may be used clinically, industrially, chemically or otherwise to reduce inflammation and to stimulate the formation of new tissue. The protein sequences used for constructing the artificial peptide included the sequences for collagen 1 and 2 (human, mouse and rat), amelogenin (human, mouse, rat, rabbit, pig and cow), ameloblastin (human, rat), bone sialoprotein (human, mouse), enamelin (human, mouse).

The artificial peptides comprised in the formulation of the current invention are particularly suitable for the induction and/or stimulation of tissue healing, as the amino acid sequences are optimized for this purpose. The use of an artificial peptide according to the invention is advantageous due to its shorter length compared to natural peptides, which facilitates the synthesis thereof and allows for the use of amino acid analogues as explained herein. Also, the use of an artificial peptide allows modifications of the amino acid sequence to enable the peptides to bind to e.g., metal surfaces or being easily purified, such as by the choice of amino acid sequences of the peptide itself or the use of N- and/or C-terminal tags.

Therefore, in one aspect, the present invention relates to a formulation comprising an artificial peptide comprising an amino acid sequence of SEQ ID NO 1 and/or SEQ ID NO 2, which is able to reduce inflammation and/or to induce and/or stimulate joint tissue healing and/or regeneration and/or for use in treating an inflamed, degenerated and/or damaged joint. Preferably such an artificial peptide consists of an amino acid sequence as shown in SEQ ID NO 1 or SEQ ID NO 2.

One embodiment of the invention relates to a formulation comprising a shorter consensus peptide sequence comprising or consisting of an amino acid sequence of Pro-X-X-Pro-Y-Y-Pro-X-X-Pro-Y-Y- Pro-X-X-Pro-Y-Y-Pro-Y-Pro-Pro-X-Pro-Pro (SEQ ID NO 2), where- in

    i) Pro is proline;
    ii) X is an amino acid selected from the group consisting of Ala, Ile, Leu, Met, Phe, Trp and Val, preferably Ile, Leu, Val and Met;
    iii) Y is an amino acid selected from the group consisting of Asn, Cys, Gln, Ser, Thr and Tyr, preferably Ser and Gln.

SEQ ID NO 2 is constructed by the assembly of amino acids 47-50, 53-66 and 70-76 of SEQ ID NO 1, i.e., amino acids underlined in SEQ ID NO 1 above. One preferred embodiment of the invention relates to a formulation comprising an artificial peptide comprising an amino acid sequence as shown in SEQ ID NO 2, more preferably consisting of an amino acid sequence as shown in SEQ ID NO 2. Another preferred embodiment relates to a formulation comprising an artificial peptide consisting of an amino acid sequence as shown in SEQ ID NO 2, which is able to reduce inflammation and/or to induce and/or stimulate joint tissue healing and/or regeneration. Another preferred embodiment relates to a formulation comprising an artificial peptide consisting of an amino acid sequence as shown in SEQ ID NO 2, which is for use in treating an inflamed, degenerated and/or damaged joint. Due to its short length, SEQ ID NO 2 is advantageous for synthetic production.

The invention also relates to a formulation according to the current invention comprising other artificial peptides with a specified amino acid sequence comprised in a consensus sequence of the invention. In a first aspect, such an artificial peptide is PLV PSY PLV PSY PLV PSY PYP PLPP (SEQ ID NO 3). Another preferred amino acid sequence is PLV PSQ PLV PSQ PLV PSQ PQP PLPP (SEQ ID NO 4). These two sequences are the two sequences of the invention that represent the most conserved sequences.

In embodiments, an artificial peptide comprised in the formulation according to the current invention comprises an amino acid sequence as shown in SEQ ID NO 4 (peptide 2 or P2) or SEQ ID NO 5 (peptide 6 or P6). As exemplified in the examples, in embodiments, an artificial peptide comprised in the formulation according to the current invention consists of an amino acid sequence as shown in SEQ ID NO 4 (peptide 2 or P2) or SEQ ID NO 5 (peptide 6 or P6).

**Table 1 Exemplified peptide sequences**

| Peptide | Peptide Sequences | Range (uM) [0.1-250 ug/mL] |
|---------|-------------------|----------------------------|
| P2 | PLV PSQ PLV PSQ PLV PSQ PQ PPLPP | 0.04-96 uM |

(continued)

| Peptide | Peptide Sequences | Range (uM) [0.1-250 ug/mL] |
|---|---|---|
| P6 | PHQ PMQ PQP PVH PMQ PLP PQ PPLPP | 0.04-90 uM |

In one aspect, the present invention relates to a formulation according to the current invention comprising an artificial peptide comprising an amino acid sequence of SEQ ID NO 4, which is able to reduce inflammation and/or to induce and/or stimulate joint tissue healing and/or regeneration. In one aspect, the present invention relates to a formulation according to the current invention comprising an artificial peptide comprising an amino acid sequence of SEQ ID NO 4, for use in treating an inflamed, degenerated and/or damaged joint. Preferably such an artificial peptide consists of an amino acid sequence as shown in SEQ ID NO 4.

In one aspect, the present invention relates to a formulation according to the current invention comprising an artificial peptide comprising an amino acid sequence of SEQ ID NO 5, which is able to reduce inflammation and/or to induce and/or stimulate joint tissue healing and/or regeneration. In one aspect, the present invention relates to a formulation according to the current invention comprising an artificial peptide comprising an amino acid sequence of SEQ ID NO 5, for use in treating an inflamed, degenerated and/or damaged joint Preferably such an artificial peptide consists of an amino acid sequence as shown in SEQ ID NO 5.

Further aspects of the invention relate to a formulation according to the current invention comprising one or more artificial peptides having between 80-100% identity with any one of the sequences of SEQ ID NO 1-5, such as peptides having 81, 82, 83, 84, 85, 86, 87, 88, 89, 90, 91, 92, 93, 94, 95, 97, 98 or 99 % identity with the sequences of SEQ ID NO1-5.

In preferred embodiments, a formulation according to the current invention comprises one or more artificial peptides disclosed herein which consist of any one of SEQ ID NO 1-5, respectively.

In a formulation according to the current invention, a peptide may further comprise N- and/or C-terminal tags comprising the amino acids His and/or Met. Met contains sulphur, which as previously explained facilitates binding to metal surfaces. His has a strong affinity for e.g., Ni and other metals. The use of these tags therefore has the advantage of enabling the peptides to attach to metal surfaces like titanium, zirconium, aluminium, tantalum, gold, surgical steel and nickel, or a metal oxide hydroxide and/or hydride surface etc. The C- and/or N-terminal tags are also useful in the process of purification of produced peptides, as is well known to the skilled person. The use of an N-terminal and/or C-terminal tag also allows the peptide to be fully exposed, i.e., the tag is used for binding the peptide to a surface and the rest of the peptide is free for interactions with e.g., atoms, molecules, cells and tissue. The use of one tag in each end of a peptide may be useful during production of the peptide, allowing one end of the peptide to be attached to a column during the purification of the peptide of interest from incomplete peptide products, while the other end of the peptide may be used for binding to a surface of interest.

Consequently, one preferred embodiment of the invention relates to a formulation according to the current invention comprising an artificial peptide as defined herein, further comprising an N-terminal and/or a C-terminal histidine tag. Such a tag may, as previously mentioned, comprise methionine and/or histidine residues, which have been attached to an artificial peptide according to the invention. In a preferred embodiment, this tag comprises 3 or more residues, such as between 3-5 or 5-10 residues. A tag can comprise any number of residues attached to an artificial peptide according to the invention, which still provides for a stable composition together with the artificial peptide according to the invention not affecting the secondary structure of the artificial peptide in a negative manner. Preferably this histidine tag consists of five histidine residues. In another preferred embodiment the artificial peptide comprises an N-terminal and/or C-terminal methionine tag, preferably consisting of five methionine residues. In another preferred embodiment, a peptide of the invention comprises a methionine tag in its C- or N-terminal end and a histidine tag in the other end.

**SEQ IDS**

In embodiments, a pharmaceutical formulation according to the current invention comprises one or more artificial peptide(s) which comprises or consists of one or more of the amino acid sequences selected from the following table (Table 2):

**Table 2 peptide sequences**

| SEQ ID NO | Peptide | Peptide Sequences |
|---|---|---|
| SEQ ID NO: 1 | | Pro-X-X-Pro-Y-Y-Y-Pro-X-X-Pro-Y-Y-Pro-X-X-Pro-X- Pro-Y-Y-Y-Y-Y-Pro-Y-Y-Y-Y-Y-Pro-X-X-Pro-X-Pro-Y-Y-Y-Pro-Y-Y-Pro-Y-Pro-X-X-Pro-Y-Pro-Y-Y-Pro-X-X- Pro-Y-Y-Pro-X-X-Pro-Y-Y-Pro-X-X-Pro-Y-Pro-Pro-X-Pro-Pro-X-X-X-X-X-X-X-Pro-X-X-Pro-X-X-X-X |

(continued)

| SEQ ID NO | Peptide | Peptide Sequences |
|---|---|---|
| SEQ ID NO: 2 | | Pro-X-X-Pro-Y-Y-Pro-X-X-Pro-Y-Y- Pro-X-X-Pro-Y-Y-Pro-Y-Pro-Pro-X-Pro-Pro |
| SEQ ID NO: 3 | | PLV PSY PLV PSY PLV PSY PYP PLPP |
| SEQ ID NO: 4 | P2 | PLV PSQ PLV PSQ PLV PSQ PQ PPLPP |
| SEQ ID NO: 5 | P6 | PHQ PMQ PQP PVH PMQ PLP PQ PPLPP |
| SEQ ID NO: 6 | P4 | PLVPSSPLVPSSPLVPSSPSPPLPP |
| SEQ ID NO: 7 | P5 | PLVPSSPLVPCCPLVPCCPSPPLPP |

In the current context, Pro is Proline (Pro); L is Leucine (Leu), V is Valine (Val), S is Serine (Ser), Q is Glutamine (Gln), H is Histidine (His), M is Methionine (Met), C is Cysteine (Cys), X is an amino acid selected from the group consisting of A (Alanine, Ala), I (Isoleucine, Ile), L (Leucine, Leu), M (Methionine, Met), F (Phenylalanine, Phe), W (Tryptophan, Trp) and V (Valine, Val), preferably Ile, Leu, Val and Met; Y is an amino acid selected from the group consisting of N (Aspargine, Asn), C (Cysteine, Cys, CysH), Q (Glutamine, Gln), S (Serine, Ser), T (Threonine, Thr) and Y (Tyrosine,Tyr), preferably Ser and Gln.

In embodiments, a pharmaceutical formulation according to the current invention comprises one or more artificial peptide(s) selected from the group consisting of artificial peptides comprising the amino acid sequence of SEQ ID NO 1, SEQ ID NO 2 SEQ ID NO 3, SEQ ID NO 4, SEQ ID NO 5, SEQ ID NO 6 and SEQ ID NO 7.

In embodiments, a pharmaceutical formulation according to the current invention comprises one or more artificial peptide(s) selected from the group consisting of artificial peptides consisting of the amino acid sequence of SEQ ID NO 1, SEQ ID NO 2 SEQ ID NO 3, SEQ ID NO 4, SEQ ID NO 5, SEQ ID NO 6 and SEQ ID NO 7.

In embodiments, a pharmaceutical formulation according to the current invention comprises one or more artificial peptide(s) selected from the group consisting of artificial peptides which are at least 90% identical to the amino acid sequence of SEQ ID NO 1, SEQ ID NO 2 SEQ ID NO 3, SEQ ID NO 4, SEQ ID NO 5, SEQ ID NO 6 and SEQ ID NO 7, respectively, such as at least 91%, 92%, 93%, 94%, 95%, 96%, 97%, 98% or 99%identical to the amino acid sequence of SEQ ID NO 1, SEQ ID NO 2 SEQ ID NO 3, SEQ ID NO 4, SEQ ID NO 5, SEQ ID NO 6, or SEQ ID NO 7, respectively.

Typically, a pharmaceutical formulation according to the current invention comprises a. 0.1-250 µg/mL, such as 0.1, 1.0, 5.0, 10, 50, 100, 200 or 250 µg/mL of an artificial peptide according to the current invention.

Typically, artificial peptides comprised in the formulations of the current invention are designed with constraints on length of no more than 30 amino acids, such as of no more than 29 amino acids, such as of no more than 28 amino acids, such as of no more than 27 amino acids, such as of no more than 26 amino acids, such as of no more than 25 amino acids, such as of no more than 24 amino acids, such as of no more than 23 amino acids, such as of no more than 22 amino acids, such as of no more than 21 amino acids, such as of no more than 20 amino acids, such as of no more than 19 amino acids, such as of no more than 18 amino acids, such as of no more than 17 amino acids, such as of no more than 16 amino acids, such as of no more than 15 amino acids, such as of no more than 14 amino acids, such as of no more than 13 amino acids, such as of no more than 12 amino acids. In addition, artificial peptides comprised in the formulations of the current invention are designed with conservation of proline positions and with systematic variation of other composing amino acids as is defined in SEQ ID NO 1 and SEQ ID NO 2.

The designed peptides (P2, P4, P5, and P6) differs in their physical, chemical, and structural properties due to the different amino acid compositions, except at the conserved proline positions in the sequence. A recently developed Web-based method of peptide design was used to explore possible correlation between the structure and biological response to the particular peptide.

### Hyaluronic acid

The current invention relates to a pharmaceutical formulation in the form of a gel comprising a. an artificial peptide characterized by a proline sequence prevalent in key matricellular proteins that partake in wound healing as well as in bone and cartilage formation and connective tissue maintenance in the majority of vertebrates, b. Hyaluronic fibres, wherein the Hyaluronic fibres may comprise crosslinked and/or non crosslinked Hyaluronic fibres.

Hyaluronic acid is a natural polymer that is natively found in the human extracellular matrix. It facilitates an environment with high water content where cells can attach and proliferate. Moreover, it is proven to exhibit intrinsic anti-bacterial and anti-inflammatory properties which are favourable for a prophylactic function. Hyaluronic acid is already used frequently for pharmacological formulations, in medical devices and in cosmetics. Therefore, it is readily available as pharma-grade raw material derived through bacterial fermentation.

Due to the unique physical and biological properties of HA (including biocompatibility and biodegradability), HA is employed in a wide range of current and developing applications. WO 03/061626 discloses an injectable, insertable, or implantable drug delivery system that form hydrogels when implanted, injected, or inserted comprising hyaluronic acid and a pharmaceutically effective compound.

Hyaluronic acid (HA) is a long-chain polysaccharide composed of repeating units of glucuronic acid and N-acetylglucosamine. It is a natural polymer with significant clinical adoption within medical fields such as ophthalmology, aesthetics (dermal fillers), and orthopaedics (viscosupplementation). The HA is naturally occurring in the body as part of the extracellular matrix which has inspired its use in medical devices, and it does indeed demonstrate high bioactivity. Initially, the hyaluronic acid was animal derived from roosters which caused the polysaccharide to be expensive and challenging to bring through the regulatory hurdles. However, the last couple of decades, hyaluronic acid production from bacterial fermentation has become the gold standard, which gives high reproducibility, low cost, and is regulatory favourable. A considerable limitation is that hyaluronic acid is rapidly cleared from the body from cleavage by the enzyme hyaluronidase, typically with a half-life of a few hours up to a couple of days. Covalently crosslinking has become a popular strategy to overcome this and to increase the therapeutic effect of the hyaluronic acid gel. A popular strategy has been to crosslink hyaluronic acid with e.g., 1,4-butanediol diglycidyl ether (BDDE) or poly(ethylene glycol) diglycidyl ether (PEGDE), where covalent bonds are formed through ring-opening esterification by means of nucleophilic attach of the reactive hydroxyl groups found in HA.

Crosslinked HA gels can also be achieved by base-catalyst reactions in an organic solvent where the carboxyl groups are activated and subsequently undergo nucleophilic acyl substitution with the hydroxyl groups. Similarly, carbodiimides such as 1-ethyl-3-(3-dimethyl aminopropyl) (EDC) can be used as a catalyst to induce crosslinking between HA polymers in a two-step process wherein the carboxylic group of the HA is first activated by the EDC in an acidic environment, then an ester bond is formed between the HA's carboxylic acid group and the hydroxyl group of the adjacent HA, and the EDC is recovered. The crosslinking can also be induced by means of photopolymerization, for instance by functionalizing the carboxylic acid groups with methacrylate end-groups.

Although there are multiple alternative strategies for crosslinking of hyaluronic acid groups, where both the carboxylic and hydroxy end-groups can be functionalized, HA crosslinked BDDE remains most popular in clinic. It has been adopted primarily for use in dermal filling.

The biomedical application of hyaluronic acid is diverse, and hyaluronic acid-based products obtain its therapeutic effect through different means. Dermal fillers require the material to fill a volume and maintain volume stability over a prolonged period. In ophthalmology hyaluronic acid can be used both to lubricate and retain water in patients prone to dry eye syndrome. In the dental domain, hyaluronic acid crosslinked with BDDE has been used to fill periodontal pockets and function as a scaffold for gingival regeneration.

Although the application and mechanism of action of the hyaluronic acid is diverse, a commonality is that it requires a high biocompatibility. Hyaluronic acid is a native component of the human extra cellular matrix and has high affinity to cells through binding with the CD44 receptors. This renders the hyaluronic acid with intrinsic anti-inflammatory properties through attenuating the release of NF-k$\beta$. When hyaluronic acid is crosslinked, this does occupy some of the carboxylic acid and hydroxide groups which will reduce its ability to bind to the CD44 receptor, thus reducing its bioactivity. Consequently, there is a trade-off between the increased physiological stability and reduction in bioactivity as the degree of crosslinking is increased. All of this is something that needs to be considered when developing a new formulation for crosslinked hyaluronic acid for biomedical application. In some recent work, poly(ethylene glycol) diglycidyl ether (PEGDE) has been used as an alternative to BDDE with results suggesting that for the same number of moles, PEGDE can give a more elastic gel with less swelling compared to BDDE, which provides a new tool for optimizing the properties of crosslinked hyaluronic acid gels.

Gels comprising or consisting of non-crosslinked Hyaluronic fibres (HA) dissolve and/or degrade faster than crosslinked HA gels. Hence peptides are released more quickly to the surrounding from a non-crosslinked HA gel than from a cross linked gel. Since the crosslinking makes the gel more stable, it will slow down the release of the peptide, as the peptide has to diffuse out of the gel, rather than being released upon degradation of the HA gel. For some applications it may be favored to obtain a fast release of the peptide and maintain the HA CD44 receptor binding, while for other applications it may be favored to obtain a slow release of the peptide form the gel. An intermediate between the two may be obtained by mixing crosslinked and non-crosslinked gels, such that a portion of the peptide is faster while another portion of the peptide is released at a slower rate.

In the current invention, it was surprisingly found that the use of hyaluronic acid increases the viscosity and/or forms a highly viscous gel-like core in the formulation comprising the artificial peptide. The crosslinking was shown to slow down the release of the artificial peptide after administration.

It is known in the art that it contributes to a better compliance if a drug can be dosed, e.g., 1-2 times per day instead of 3-4 times per day. However, it may also be favourable that there is an immediate release of at least a portion of the drug followed by a sustained release of the drug.

In the current context, "Hyaluronic acid" (HA) is defined herein as an un-sulphated glycosaminoglycan composed of repeating disaccharide units of N-acetylglucosamine (GlcNAc) and glucuronic acid 20 (GlcUA) which are linked together by alternating beta-1,4 and beta-1,3 glycosidic bonds. Hyaluronic acid is also known as hyaluronan, hyaluronate, or HA. The terms hyaluronan, hyaluronic acid, and HA are in the current context used interchangeably.

The level of hyaluronic acid may be determined according to the modified carbazole method (Bitter and Muir, 1962, Anal Biochem. 4: 330-334).

In a preferred embodiment the hyaluronic acid used according to the invention is of a very pure quality, in particular current Good Manufacturing Practice (cGMP) quality.

The hyaluronic acid used in the formulations according to the current invention can be derived from a variety of sources. Rooster combs are a significant commercial source for HA. Alternatively, microorganisms are an alternative source. US 4,801,539 and EP 0694616 disclose fermentation methods for preparing hyaluronic acid involving a strain of *Streptococcus zooepidemicus.*

In embodiments, the hyaluronic acid or salt thereof is of microbial origin, preferably recombinantly produced. In one embodiment, the hyaluronic acid or salt thereof is produced by a Gram-positive bacterium, such as, but not limited to *Bacillus and/or Streptococcus.* In a currently preferred embodiment, the hyaluronic acid or salt thereof is produced by *Bacillus subtilis.* The hyaluronic acid or salt thereof may be produced as disclosed in WO 03/054163.

Hyaluronan synthases have been described from vertebrates, bacterial pathogens, and algal viruses (DeAngelis, P. L., 1999, Cell. Mol. Life Sci. 56: 670-682). WO 99/23227 discloses a Group I hyaluronate synthase from *Streptococcus equisimilis.* WO 99/51265 and WO 00/27437 describes a Group II hyaluronate synthase from *Pasturella multocida.* Ferretti et al. disclose the hyaluronan synthase operon of *Streptococcus pyogenes,* which is composed of three genes, *hasA, hasB,* and *hasC,* that encode hyaluronate synthase, UDP glucose dehydrogenase, and UDP-glucose pyrophosphorylase, respectively (Proc. Natl. Acad. Sci. USA. 98, 4658-4663, 2001). WO 99/51265 describes a nucleic acid segment having a coding region for a *Streptococcus equisimilis* hyaluronan synthase.

The host cell may be any *Bacillus* cell suitable for recombinant production of hyaluronic acid. The *Bacillus* host cell may be a wild-type *Bacillus* cell or a mutant thereof. *Bacillus* cells useful in the practice of the present invention include, but are not limited to, *Bacillus agaraderhens, Bacillus alkalophilus, Bacillus amyloliquefaciens, Bacillus brevis,* 20 *Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells. Mutant *Bacillus subtilis* cells particularly adapted for recombinant expression are described in WO 98/22598. Non encapsulating *Bacillus* cells are particularly useful in the present invention. Since the hyaluronan of a recombinant *Bacillus* cell is expressed directly to the culture medium, a simple process may be used to isolate the hyaluronan from the culture medium.

*Salts of hyaluronic acid*

Any salt of hyaluronic acid may be used according to the present invention.

In a preferred embodiment the salt of hyaluronic acid is an inorganic salt, preferably sodium hyaluronate, potassium hyaluronate, ammonium hyaluronate, calcium hyaluronate, magnesium hyaluronate, zinc hyaluronate, or cobalt hyaluronate.

*HA Molecular weight*

Any molecular weight may be used according to the invention, but in a preferred embodiment the hyaluronic acid or the salt thereof has an average molecular weight in the range of 0.2-6 MDa; more preferably an average molecular weight in the range of 0.7-4.0 MDa; more preferably an average molecular weight in the range of 1.0-3.0 MDa; and even more preferably an average molecular weight in the range of 1.25 to 1.75 MDa. The molecular weight may be determined as known in the art.In preferred embodiments, the molecular weight of the hyaluronic acid is about 1.5 MDa.

The specific MWs of the HA In the currently described formulation are chosen to provide a beneficial viscosity relationship, i.e., a slow release due to higher entanglement as is described in e.g., Falcone 2005, as well as in WO 2013/030348 A1, showing experiments of increasing HA concentrations vs release times.

A pharmaceutical formulation according to the current invention comprises cross-linked Hyaluronic fibres (HA-XL), and/or non-crosslinked Hyaluronic fibres (HA).

Typically, a pharmaceutical formulation according to the current invention comprises 1-40 mg/mL, such as 1.0, 2.5, 4, 10, 20, 25 or 40 mg/mL cross-linked Hyaluronic fibres (HA-XL), and/or 1-40, such as 1.0, 2.5, 4, 10, 20, 25 or 40 mg/mL mg/mL non-crosslinked Hyaluronic fibres (HA).

A pharmaceutical formulation according to the current invention can comprise cross-linked Hyaluronic fibres (HA-XL) which comprise or consist of 1,4-butanediol diglycidyl ether cross-linked Hyaluronic fibres (HA-BDDE) and/or poly(ethylene glycol) diglycidyl (PEGDE) ether cross-linked Hyaluronic fibres (HA-PEGDE).

Preferably, the formulation of the current invention comprises both crosslinked and non-crosslinked HA. The ratio of crosslinked to non-crosslinked HA may be varied for specific purposes. Accordingly, in embodiments, the formulation of the current invention may comprise crosslinked and non-crosslinked HA, wherein 87.5-92.5%, such as about 90% of the HA is crosslinked and between 7.5% and 12.5%, such as about 10% of the HA is non-crosslinked.

In a pharmaceutical formulation according to the current invention, the Hyaluronic acid fibres are typically between 0.7-4.0 MDa, such as between 1.0-2.0, between 1.5-1.8, or between 3.0-3.3 MDa. In one embodiment the Hyaluronic acid monomers are between 3.0-3.3 MDa.

In a currently preferred embodiment, a pharmaceutical formulation according to the current invention comprises a. 0.1-250ug/mL such as 50 $\mu$g/mL of an artificial peptide and b. 1-40 mg/mL Hyaluronic fibres (HA).

In a currently preferred embodiment, a pharmaceutical formulation according to the current invention comprises a. 50 $\mu$g/mL of an artificial peptide, b. 20 mg/mL 1,4-butanediol diglycidyl ether cross-linked Hyaluronic fibres (HA-BDDE), and c. 2.5 mg/mL non-crosslinked Hyaluronic fibres (HA).

In a currently preferred embodiment, a pharmaceutical formulation according to the current invention comprises a. 50 $\mu$g/mL of an artificial peptide, b. 20 mg/mL poly(ethylene glycol) diglycidyl (PEGDE) ether cross-linked Hyaluronic fibres (HA-PEGDE), and c. 2.5 mg/mL non-crosslinked Hyaluronic fibres (HA). Preferably the, poly(ethylene glycol) diglycidyl ether has a molecular weight (MW) of 250-1500 Da, such as about 500 Da.

In a currently preferred embodiment, a pharmaceutical formulation according to the current invention comprises a. 50 $\mu$g/mL of an artificial peptide with the amino acid sequence of SEQ ID NO 4 and/or SEQ ID NO 5, b. 20 mg/mL 1,4-butanediol diglycidyl ether cross-linked Hyaluronic fibres (HA-BDDE), and c. 2.5 mg/mL non-crosslinked Hyaluronic fibres (HA).

In a currently preferred embodiment, a pharmaceutical formulation according to the current invention comprises a. 50 $\mu$g/mL of an artificial peptide with the amino acid sequence of SEQ ID NO 4 and/or SEQ ID NO 5, b. 20 mg/mL poly(ethylene glycol) diglycidyl (PEGDE) ether cross-linked Hyaluronic fibres (HA- PEGDE), and c. 2.5 mg/mL non-crosslinked Hyaluronic fibres (HA).

**A pharmaceutical formulation**

The current invention relates to a pharmaceutical formulation in the form of a gel comprising a. an artificial peptide characterized by a proline sequence prevalent in key matricellular proteins that partake in wound healing as well as in bone and cartilage formation and connective tissue maintenance in the majority of vertebrates, b. cross-linked Hyaluronic fibres (HA-XL), and/or c. non-crosslinked Hyaluronic fibres (HA) and in particular to its uses in medicine, such as for use in treating an inflamed, degenerated and/or damaged joint, such as for use in the treatment of arthritis.

A pharmaceutical formulation according to the current invention is stable/in gel form at RT for at least 1-2 years and/or in the body for at least 30 days.

A pharmaceutical formulation according to the current invention is stable/in gel form at RT for at least 1-2 years, such as for at least 1 year, such as for at least 1.5 years, such as for at least 1.7 years, such as for at least 2 years, such as for 1 year, 1.5, 1.7, 1.8, 2, 3, or 5 years.

A pharmaceutical formulation according to the current invention is stable/in gel in the body for at least 30 days, such as for at least 31 days, such as for at least 32 days, such as for at least 35 days, such as for at least 40 days, such as for at least 50 days, such as for at least 60 days, such as for at least 90 days.

In one aspect, a pharmaceutical formulation according to the current invention releases the comprised artificial peptide at a controlled rate. In the current context, the term "at a controlled rate" is used to describe the release to not be a burst release. Controlled release is herein characterized by releasing drugs according to a predictable and rational programmed rate to achieve the optimal target-drug concentration. This dosage form enhances the safety, efficacy, reliability, and convenience of drug therapy. Although this is a slow releasing system, unlike sustained release, this process is designed to produce predictable, constant concentrations of the drug. For this approach, the concentration of the active ingredient in the target tissue is controlled, not just the release of the drug.

In one aspect, a pharmaceutical formulation according to the current invention releases the comprised artificial peptide at a controlled rate, such as of about 2 ug per hour, such as at the most 10, 5, 4, 3, 2, 1.5, 1.2 or 1 ug per hour or such as about 10ng, 100ng, 500ng per hour, or such as 0.01, 0.1, 1, 2, 3, 4, 5, 6, 7, 8, 9 or 10 ug per hour. In one aspect, a pharmaceutical formulation according to the current invention releases the comprised artificial peptide at a controlled rate, such as of between 0.1-10ug per hour, such as of between 1-8ug per hour, such as of between 1.5-5ug per hour.

A pharmaceutical formulation according to the current invention can further comprise one or more sources of fluoride, such as e.g., one or more fluoride sources selected from the group consisting of NaF, CaF2 and ZnF2. Preferably the fluoride source is NaF. In further embodiments a pharmaceutical formulation according to the current invention does not comprise one or more sources of fluoride.

A pharmaceutical formulation according to the current invention can further comprise one or more of a component selected from the group consisting of: Sorbitol, Xylitol, NaOH, HCL, Phosphate Buffer Solution (PBS) and acetic acid.

In an embodiment, the pharmaceutical formulation/composition comprises Sodium fluoride and/or Citric Acid. In further embodiments, the pharmaceutical formulation/composition does not comprise sodium fluoride and/or citric acid.

In preferred embodiments, the pharmaceutical formulation/composition comprises a. 50 $\mu$g/mL of an artificial peptide with the amino acid sequence of SEQ ID NO 4 and/or SEQ ID NO 5, b. 20 mg/mL 1,4-butanediol diglycidyl ether and/or poly(ethylene glycol) diglycidyl ether cross-linked Hyaluronic fibres (HA-BDDE or HA-PEGDE), c. 2.5 mg/mL non-crosslinked Hyaluronic fibres (HA), d. 97 wt.% water, e. 0.11 wt.% Sodium Hydroxide, f. 0.3 wt.% Sodium Chloride, g. 0.36 wt.% Disodium Phosphate and h. 0.08 wt.% Sodium Phosphate.

## Osmolarity

Maintaining an appropriate osmolarity ensures compatibility with the body's cells and prevents cellular damage, and solutions with similar osmolarity to body fluids reduce tissue irritation, minimize inflammation and other complications. Accordingly, maintaining a suitable osmolarity of the pharmaceutical formulation/composition of the current invention is highly beneficial. Many buffers, such as PBS, mimic the preferred osmolarity of mammals, and have an osmolarity of about 280-310 mOsm/L. Accordingly, in embodiments, the pharmaceutical formulation of the present invention has an osmolarity of 50-400 mOsm/L, such as between 100 and 310 mOsm/L, or such as between 125 and 175 mOsm/L, such as about 150 mOsm/L, or such as between 275 and 325 mOsm/L, such as about 300 mOsm/L.

## Citric Acid

Citric Acid is used for product preservation and for pH adjustment of the pharmaceutical formulation/composition according to the current invention. The citric acid has an impact on the stability of the product. Citric acid can create an acidic environment that can inhibit bacterial growth and reduce the risk of spoilage. This is because most bacteria prefer a neutral or slightly alkaline environment and cannot survive in highly acidic conditions. Citric acid can also chelate, or bind to, certain essential nutrients like iron, which some bacteria require for growth.

## Active ingredient

In embodiments, a pharmaceutical formulation according to the current invention can further comprise another/-additional active ingredient, such as, but not limited to, an active ingredient selected from the group consisting of growth factors, plasma rich fibrin/plasma and enamel matrix derivatives.

## Medical uses

In the experimental section, a pharmaceutical formulation according to the current invention is shown to have beneficial effects on joint tissue healing and/or regeneration, which is highly beneficial in the treatment of an inflamed, degenerated and/or damaged joint.

Surprising, this beneficial effect was not only seen on the soft tissue healing, in or adjacent to the joint, such as on muscles, tendons, ligaments, and other soft tissues, but also on cartilage healing and restoration. In the process of joint tissue healing in particular cartilage healing it is not easily affected or assisted. Under normal conditions, cartilage healing is limited due to the avascular nature of cartilage, resulting in the formation of fibrocartilage that does not fully restore the original tissue's structure and function. The current disclosure for the first time demonstrates that a pharmaceutical formulation according to the current invention is effective in joint tissue healing and/or regeneration in all tissues in the inflamed, degenerated and/or damaged joints., and in particular in cartilage regeneration in said joints.

Thus, it was surprising that the pharmaceutical formulation described herein effectively heals and/or regenerates both soft tissue and cartilage in inflamed, degenerated and/or damaged joints. In addition, as a secondary effect from the reduced inflammation as well as the healing and/or regeneration of both soft tissue and cartilage in inflamed, degenerated and/or damaged joints, the pharmaceutical formulation described herein also effectively reduced inflammation in hard tissue adjacent to the treated joint, such as the bone.

Joint inflammation is a common condition characterized by the swelling and pain in one or more joints. It can arise from various factors such as injury, infection, inflammation and/or autoimmune disorders. If left untreated, chronic inflammation can lead to joint damage, causing irreversible changes to the joint structure and function.

One aspect of the current invention thus relates to the novel pharmaceutical formulation according to the current invention for use in tissue healing and/or regeneration in inflamed, degenerated and/or damaged joints., such as, but not limited to, use in the treatment of arthritis, preferably rheumatoid arthritis and/or osteoarthritis, wherein it may e.g., be used as viscosupplementation.

A pharmaceutical formulation according to the current invention may for instance be for use in stimulating collagen production, and/or for promoting oriented collagen formation in joint tissue healing.

The novel pharmaceutical formulation according to the current invention can, further or in combination with the above-mentioned uses, be for use in an anti-inflammatory and/or antimicrobial treatment, such as e.g., but not limited to, or use in preventing, ameliorating, prevent progression of and/or treating arthritis and/or osteomyelitis.

*Osteomyelitis*

Osteomyelitis is a serious and potentially life-threatening infection that affects hard-tissue such as e.g., bones. It occurs when bacteria or other infectious agents invade the bone tissue, leading to inflammation, destruction of bone, and the formation of abscesses.

Osteomyelitis can occur in any bone in the body, but it most commonly affects the long bones in the arms (humerus, radius, ulna, metacarpals, and phalanges) and legs (femur, tibia, fibula, metatarsals, and phalanges), particularly in children, as well as the vertebrae in the spine. The infection can be acute, developing rapidly and causing severe symptoms, or chronic, with a slower onset and more persistent course.

The majority of osteomyelitis cases are caused by bacteria, with *Staphylococcus aureus* being the most common culprit. Other bacteria, such as *Streptococcus* and *Escherichia coli,* can also be responsible.

Osteomyelitis can also occur when bacteria from other parts of the body spread through the bloodstream and reach the bone, a condition known as hematogenous osteomyelitis. Osteomyelitis may also develop as a result of a direct injury to the bone, such as an open fracture or a surgical procedure. In these cases, bacteria can enter the bone through the open wound.

As is shown in the examples, it was surprisingly found that the pharmaceutical formulation disclosed herein, may have beneficial bacteriostatic effects, particularly on *Staphylococcus aureus.* Such bacteriostatic effects are highly beneficial in the treatment of osteomyelitis, where the underlying reason is a bacterial infection in the hard tissue. In particular the pharmaceutical formulation of the present invention is suitable for such treatment since hyaluronic acid is endogenous to the human body and it is unlikely that HA provoke any harmful immunological response following its administration. Furthermore, the combination of sustained release of the synthetic peptide and the hyaluronic acid matrix ensures that the treatment will be prolonged and effective over a longer period of time. In addition, the pharmaceutical formulation may also be supplemented with additional active pharmaceutical ingredients, such as one or more antimicrobial agents, such as e.g., vancomycin, ceftriaxone, clindamycin, rifampin and/or fluoroquinolones.

One of the benefits of the novel pharmaceutical formulation as disclosed herein for use in the treatment of osteomyelitis is that the gel like formulation is less likely to mitigate following injection, due to the higher viscosity. This is highly beneficial since a formulation which is not gel-like would allow for the synthetic peptide to mitigate away from the injection area much faster than it would from the gel-like formulation. In addition, the formulation of the peptide in the gel-like phase also inhibits the protease mediated degradation. Thus, there are several benefits associated with the use of the herein disclosed pharmaceutical formulation in osteomyelitis.

Accordingly, in aspects the novel pharmaceutical formulation according to the current invention may be for use in the treatment of osteomyelitis. In addition, the novel pharmaceutical formulation according to the current invention may be for use in alleviating bone pain associated to osteomyelitis and/or for use in reducing bone inflammation in osteomyelitis. in aspects the novel pharmaceutical formulation according to the current invention may be for use in reducing and/or suppressing an infection and/or inflammation in a joint and/or in tissue adjacent to a joint.

*Arthritis*

Arthritis is a chronic condition characterized by inflammation and stiffness of the joints, leading to pain and reduced mobility. It encompasses various forms, including osteoarthritis and rheumatoid arthritis. Arthritis often affects older adults but can also occur in younger individuals, where the onset of arthritis is often a result of trauma to the joint, such as damage from surgery, or joint injury from e.g., sports and/or athletics.

It can impact multiple joints throughout the body predominantly knees, shoulders, elbows, and hips, but also commonly seen in fingers, wrist and toes causing discomfort and limitations in daily activities, requiring ongoing treatment and care.

Accordingly, the anti-inflammatory properties of a pharmaceutical formulation of the present invention makes the formulation especially suited for treatment or supplementary treatment of arthritis, but also for preventing, ameliorating and/or prevent the progression of arthritis.

*Rheumatoid arthritis*

Rheumatoid arthritis (RA) is a chronic autoimmune disorder that primarily affects the joints, causing inflammation and pain in the joint tissue. The immune system mistakenly attacks the body's own tissues, particularly the synovium (lining of the joints), leading to joint swelling, stiffness, and deformity. RA commonly affects small joints in the hands and feet but can involve larger joints as well. It is characterized by periods of flare-ups and remission. Besides joint symptoms, RA may cause fatigue, fever, weight loss, and affect other organs.

Accordingly, the anti-inflammatory properties of a pharmaceutical formulation of the present invention makes the formulation especially suited for treatment or supplementary treatment of Rheumatoid arthritis.

*Osteoarthritis*

Osteoarthritis is a degenerative joint disease that primarily affects the cartilage in joints. It is the most common form of arthritis and often occurs as a result of wear and tear on the joints over time but may also arise from acute injury to the joint. While it can affect any joint in the body, it commonly affects the knees, hips, hands, and spine but is also prevalent in fingers, wrist and toes.

In a healthy joint, cartilage provides a smooth surface that allows for frictionless movement. However, in osteoarthritis, the cartilage gradually wears away, leading to joint damage and inflammation. As the disease progresses, the joint may develop bony growths called osteophytes or bone spurs, which further contribute to pain and limited mobility. Typically, individuals with osteoarthritis experience pain during or after movement. Initially, the pain may be mild and intermittent, but it can worsen over time. Inflammation of the joint can cause localized swelling or tenderness, which may contribute to the joint's warm and red appearance.

The anti-inflammatory properties of a pharmaceutical formulation of the present invention makes the formulation especially suited for treatment or supplementary treatment of osteoarthritis but also for preventing, ameliorating and/or prevent the progression of osteoarthritis. This is in part due to the synergistic effect between the hyaluronic acid and the synthetic peptides which effectively reduces the inflammation in the joint tissue e.g., cartilage, an effect which the inventors envisioned also affects the surrounding soft and hard tissue, overall providing an anti-inflammatory effect in the joint, which reduces pain, swelling and/or tenderness.

*Viscosupplementation*

Viscosupplementation is a medical procedure used to treat symptoms associated with osteoarthritis, particularly e.g., in the knee and/or elbow joint. In general, viscosupplementation involves the injection of a gel-like formulation of hyaluronic acid (HA) directly into the affected joint. HA normally acts as a lubricant and shock absorber in the joints, and injection of HA may restore the mobility of the joint by restoring the cartilage in the joint. Accordingly, the novel pharmaceutical formulation according to the current invention may be for use in viscosupplementation.

During viscosupplementation, a healthcare professional administers a series of injections into the knee joint space. The procedure aims to supplement the diminished levels of hyaluronic acid in the joint affected by osteoarthritis, thereby improving joint function and reducing pain. In addition, the beneficial function of the synthetic peptides disclosed herein may further add to the pain reduction due to the anti-inflammatory effects of the peptides. To enhance the effects of viscosupplementation with a pharmaceutical formulation as disclosed herein, it is highly beneficial that the formulation comprises crosslinked HA, which releases the peptide over a longer period of time, while also exerting the lubricant and shock absorption function of HA for a longer period of time. This slowed release ensures a longer effect of the viscosupplementation, compared to non-cross-linked HA.

Accordingly, the injected pharmaceutical formulation as disclosed herein may act as a viscoelastic agent, providing lubrication and cushioning within the joint, where it e.g., helps to reduce friction between the bones, allowing for smoother movement and reducing pain during joint motion.

Additionally, pharmaceutical formulation disclosed herein further have anti-inflammatory properties, which can further alleviate pain and swelling associated with osteoarthritis, which may be treated via. viscosupplementation.

Viscosupplementation is typically recommended for individuals who have not responded adequately to conservative treatment options such as pain medications, physical therapy, or corticosteroid injections. It is most commonly used for the treatment of knee osteoarthritis but may also be used in other joints affected by osteoarthritis, such as the hip, elbow, or shoulder.

The number of injections administered and the interval between injections can vary depending on the specific product used and the individual's condition. Typically, a series of three to five injections is given over a period of several weeks such as e.g., 1, 2, 3, 4, ,5 ,6, 7, 8, 9, 10, 15, 20, 26, 30, 40, 50 or 52 weeks, or between 1-52 weeks.

For instance, patients who have undergone meniscectomy may have an increased benefit of viscosupplementation, due to positive effects on the modulation of the entire joint environment, such as reduce pain, reduced swelling, and faster functional recovery following meniscectomy.

*Regeneration* of *cartilage tissue*

Cartilage is a connective tissue found throughout the body, providing a smooth surface for joint movement and acting as a cushion between bones. Various medical conditions can affect cartilage, leading to significant health issues.

The pharmaceutical formulation disclosed herein may also be used in the treatment of cartilage tears, such as meniscal tears in the knee or labral tears in the shoulder. Cartilage tears occur when the cartilage in a joint is damaged or torn. This can result from traumatic injuries or repetitive stress on the joint, leading joint inflammation, which is often associated with joint pain, swelling, and reduced joint function. Accordingly, the pharmaceutical formulation disclosed herein may also be used in the treatment of chondromalacia patellae. Chondromalacia patellae is a condition where the cartilage on the underside of the kneecap (patella) becomes soft and damaged. It often causes pain and a grinding sensation in the knee joint, especially during activities that involve bending the knee.

Accordingly, the pharmaceutical formulation for use as disclosed herein may also be used in the treatment of induce and/or support tissue healing and/or regeneration in a joint and/or in tissue adjacent to a joint.

**Medical treatment**

In another aspect the invention relates to treating a patient suffering from a disease, disorder and/or condition in joint tissue by administering a pharmaceutical formulation according to the current invention and thus inducing in said patient reduction of inflammation, joint tissue healing and/or regeneration, collagen production, and/or oriented collagen formation.

The invention also relates to treating a patient suffering from a disease, disorder and/or condition resulting in joint inflammation by administering a pharmaceutical formulation according to the current invention as an anti-inflammatory and/or antimicrobial treatment.

The invention also relates to treating a patient suffering, or suspected of suffering from a disease, disorder and/or condition resulting in joint inflammation by administering a pharmaceutical formulation according to the current invention.

The invention also relates to treating a patient having undergone surgery resulting in joint inflammation by administering a pharmaceutical formulation according to the current invention, as an anti-inflammatory and/or antimicrobial treatment and/or prophylactic treatment. Accordingly, the invention also relates to treating a patient having undergone surgery resulting in joint inflammation by administering a pharmaceutical formulation according to the current invention, in order to prevent, ameliorate, and/or prevent progression of joint inflammation, degradation and/or damage.

The invention also or in combination relates to treating a patient suffering from a disease, disorder and/or condition resulting in joint inflammation by administering a pharmaceutical formulation according to the current invention to said patient.

In one embodiment, the invention relates to the use of the pharmaceutical composition for the preparation of a medicament for the treatment of a disease, disorder and/or condition resulting in joint inflammation and/or degeneration, such as for reducing inflammation, inducing and/or stimulating tissue healing and/or regeneration in inflamed and/or damaged joints. In particular, the invention relates to the use of a pharmaceutical composition according to the current invention for the preparation of a medicament for the treatment of arthritis, such as e.g., osteoarthritis and/or rheumatoid arthritis.

In one aspect, the invention also relates to the use of a pharmaceutical composition according to the current invention for the preparation of a medicament for regenerating inflamed and/or damaged joint tissue, preferably connective tissue, such as e.g., cartilage tissue.

In one aspect, the invention also relates to the use of a pharmaceutical composition according to the current invention for the preparation of a medicament for use in an anti-inflammatory and/or antimicrobial treatment.

In one aspect, the invention also relates to the use of a pharmaceutical composition according to the current invention for the preparation of a medicament for use in a regenerative treatment.

In one aspect, the invention also relates to the use of a pharmaceutical composition according to the current invention for the preparation of a medicament for use as a prophylactic.

In one aspect, the invention also relates to the use of a pharmaceutical composition according to the current invention for the preparation of a medicament for treating a patient suffering from osteoporosis, rheumatoid arthritis and/or osteoarthritis.

In one aspect, the invention also relates to the use of a pharmaceutical composition according to the current invention for the preparation of a medicament for use in viscosupplementation.

**Administration**

A pharmaceutical formulation of the invention may be administered to a subject in need thereof by any suitable route depending on the tissue which the peptide is to be administered to, for example by topical (dermal), local, and/or surgical administration, and/or by injection.

In particular, application to or into the joint, or to the tissue adjacent to the joint is of great importance.

Furthermore, a composition may be adapted to administration in connection with surgery, e.g., in connection with incision within the body. The pharmaceutical formulation of the invention may also be administered via local injections, by application in a gel or via a medical device, such as a medical prosthetic device, e.g., a graft, scaffold or bioglass material.

In one aspect, a pharmaceutical formulation of the invention is administered as a minimally invasive administration by injection.

In one embodiment, a pharmaceutical formulation of the invention is provided in single use units in syringes for effective delivery in the needed region.

The current invention thus in one aspect relates to the use of a pharmaceutical composition according to the current invention for local application to a joint, or to the tissue adjacent to a joint.

The current invention thus in one aspect relates to the use of a pharmaceutical composition according to the current invention administered as a minimally invasive administration by injection.

**Method for producing a pharmaceutical formulation**

The current invention also relates to methods for producing a pharmaceutical formulation as disclosed herein. Accordingly, further aspects of the invention relate to a method for producing a pharmaceutical formulation according to the current invention, said method comprises, a. providing an artificial peptide as defined herein, b. providing hyaluronic acid (HA) with a molecular weight of 0.7-4 MDa, such as between 1.0-2.0, between 1.5-1.8, or between 3.0-3.3 MDa, such as 1.5MDa, c. mixing 0.1-250 $\mu$g/mL of said artificial peptide and 1-40 mg/mL of said HA and d. optionally, adding a source of fluoride to said mixture, and wherein, said pharmaceutical formulation has an osmolarity of 50-400 mOsm/L, such as between 100 and 310 mOsm/L, or such as between 125 and 175 mOsm/L, such as about 150 mOsm/L, or such as between 275 and 325 mOsm/L, such as about 300 mOsm/L. In additional embodiments, the said Hyaluronic acid provided in step b. of said method comprises or consists of BDDE and/or PEGDE crosslinked hyaluronic acid.

In additional embodiments, said method may comprise a. providing the artificial peptide at a concentration of up to 100 mg/mL, b. adding Hyaluronic acid fibres dissolved at a concentration of 10 wt.% in 0.3M NaOH, c. mixing in a cross-linking agent such as e.g., BDDE and/or PEGDE, d. heating the reaction vessel to 20-100 °C and incubating for between 1-2 hours to induce gelification, e. cooling and neutralizing the solution, f. homogenizing the gel, g. dialyzing the gel for at the least 18 hours in sterile PBS, h. adding non-crosslinked HA in an amount of about 10% of the crosslinked HA, i. homogenizing the formulation to reassure uniform distribution of the peptide, and j. optionally adding Phosphate Buffer Solution (PBS).

In embodiments, a method for producing a pharmaceutical formulation according to the current invention, the artificial peptide in step a. can be dissolved in 1% acetic acid at a concentration of 10 mg/mL, followed by b. adding Hyaluronic acid fibres (3.0-3.3 MDa) dissolved at a concentration of 10 wt.% in an alkaline solution, preferably NaOH, c. admixing 1,4-butanediol diglycidyl ether (BDDE) and/or poly(ethylene glycol) diglycidyl (PEGDE), d. heating the reaction vessel to about 40 °C and incubating for approximately 4 hours, e. cooling and neutralizing the solution with an acidic solution, preferable HCl, f. homogenizing the gel into particles of 100-400 $\mu$m. In embodiments, the method further comprises g. dialyzing the gel in a biocompatible solution such as e.g., PBS or saline, h. optionally, adding non-crosslinked HA, in an amount of about 10 wt.% of the crosslinked HA), i. homogenizing the formulation to reassure uniform distribution of the peptide, and j. optionally adding Phosphate Buffer Solution (PBS).

In one embodiment, the production process comprises:

1. Dissolving artificial peptides in distilled water to a final concentration of about 0.1-250 ug/mL, 1-10mg/mL, or of at least 0.1ug/mL.
2. Mixing by vertexing,
3. Creating an aqueous solution containing water for injection, NaCl, $Na_2HPOa$, $NaH_2PO_4$, and artificial peptides,

such as P2 and P6, and optionally citric acid.

4. Adding NaOH for pH adjustment,

3. Adding sodium hyaluronate,

4. Optionally adding a crosslinking agent such as BDDE and/or PEGDE,

5. Mixing for 24h under gentle agitation,

6. Filling into one or more syringe(s),

7. Packing into pouch,

8. Autoclaving the packaged product.

In embodiments, the syringe has a volume of about 0.5-1.5 mL.

Accordingly, the current invention also relates to a kit for clinical use comprising a pouch, wherein said pouch comprises one or more single use syringes prefilled with a pharmaceutical formulation as disclosed herein, and instructions for use.

Due to their shorter length, compared to natural proteins, the artificial peptides comprised in the pharmaceutical formulation according to the current invention are easier to produce, e.g., by synthetic production or biosynthesis. The artificial peptides of the invention may be produced by any known method for production of peptides, such as synthetic production by chemical synthesis. Synthetic production also allows the use of amino acid analogues which may improve the stability of the peptides produced. The skilled person knows what methods are available for the synthesis of an amino acid sequence.

Preferably, bioproduction may be used as a method for producing the peptides. Bioproduction means the production of an amino acid sequence in a biological system, such as a cell culture or in microbial cells, e.g., bacterial cells. For bioproduction, it is necessary to construct the corresponding nucleic acid sequence encoding a specific amino acid sequence. The skilled person readily knows how to construct such a nucleic acid sequence once a specific amino acid sequence to be synthesized is determined upon, and how to produce the peptide and purify it from the system used to produce it (see e.g. Svensson J, Andersson C, Reseland JE, Lyngstadaas SP, Bulow L. Histidine tag fusion increase expression levels of active recombinant Amelogenin in Escherichia coli. Protein Expr Purif, 48; 134-41 (2006)).

In one embodiment, the product is packed into glass syringes, such as but not limited to the BD Hylok™ pre-fillable glass syringe in which it is also autoclaved to mitigate for bacterial contamination. Prior to autoclaving, it can be packed into a E-line pouch to maintain the aseptic condition of the product. Along with the syringe with the gel, there can also be packed an application tip (19G, blunt) inside the pouch.

When delivered in a single use pre-filled syringe (e.g., at a 1 mL volume, or 0.7 mL volume), this is to allow it to be efficiently used in dental clinics. To prevent cross contamination between users, each package consists of e.g., 2, 4 or up to 8 single use units with accompanying dispensing tips. The syringe and dispensing tips are packed together in a single pouch which is packed in aseptic conditions.

The product can be a sterile or a non-sterile product.

**A pharmaceutical formulation obtained by a method according to the current invention**

In one aspect, the current invention relates to a pharmaceutical formulation obtained by a method according to the current invention, as well as to its use as a medicament.

The invention also relates to a pharmaceutical formulation comprising an artificial peptide as defined herein or a combination of two or more artificial peptides as defined herein. Such a pharmaceutical formulation optionally also comprises a pharmaceutically acceptable carrier, excipient and/or diluent.

Compositions in the present context embrace pharmaceutical and cosmetic compositions as well as compositions belonging to the so-called grey area between pharmaceuticals and cosmetics, namely cosmeceuticals.

The pharmaceutical composition is typically in the form of a gel, preferably in the form of a hydrogel.

In one aspect, a pharmaceutical formulation of the invention is formulated for minimally invasive administration by injection.

In one aspect, a pharmaceutical formulation of the invention is formulated for local application. In another aspect, a pharmaceutical formulation of the invention is formulated for delivery by dermal application, topical application and/or transdermal patches.

The compositions may be formulated according to conventional pharmaceutical practice, see, e.g., "Remington's Pharmaceutical Sciences" and "Encyclopedia of Pharmaceutical Technology", edited by Swarbrick, J. & J. C. Boylan, Marcel Dekker, Inc., New York, 1988.

A pharmaceutically or cosmetically acceptable excipient, carrier and/or diluent is a substance which is substantially harmless to the individual to which the composition is to be administered. Such an excipient, carrier and/or diluent normally fulfils the requirements given by the national health authorities. Official pharmacopoeias such as e.g., the British Pharmacopoeia, the United States of America Pharmacopoeia and The European Pharmacopoeia set standards for pharmaceutically acceptable excipients.

Whether a pharmaceutically acceptable excipient is suitable for use in a pharmaceutical composition is generally dependent on which kind of dosage form is chosen. In the following are given examples of suitable pharmaceutically acceptable excipients for use in different kinds of compositions for use according to the invention.

The choice of pharmaceutically acceptable excipient(s) in a composition for use according to the invention and the optimum concentration thereof cannot generally be predicted and must be determined on the basis of an experimental evaluation of the final composition. However, a person skilled in the art of pharmaceutical formulation can find guidance in e.g., "Remington's Pharmaceutical Sciences", 18th Edition, Mack Publishing Company, Easton, 1990.

The concentration of the artificial peptide in a pharmaceutical composition according to the invention will, as the skilled person readily understands, vary depending on the intended use of the composition. Typically, the concentration of the peptide in the pharmaceutical composition is about 0.01-1 mg/mL. The amount applied *in vivo* to a subject is typically about 10 ng/cm$^2$ to 0.1 mg/cm$^2$, preferably about 1 ug/cm$^2$.

In one embodiment, a pharmaceutical composition according to the current invention comprises the following ingredients as listed in table 3.

*Table 3. Exemplary ingredient list in descending order after content in a final pharmaceutical composition according to the current invention, supplier of the ingredient, purity grade and weight percentage.*

| Ingredient list | wt.% Content |
| --- | --- |
| Water | 97% |
| Sodium Hyaluronic Acid IV (m3/kg) =2.1 | 2.0% |
| Citric Acid | 0.2% |
| NaOH | 0.11% |
| NaCl | 0.3% |
| Na$_2$HPO$_4$, 12H$_2$O | 0.36% |
| NaH$_2$PO$_4$, 2H2O | 0.08% |
| Peptide (1:1 mix P2&P6) | 2 $\mu$g/mL |

*Table 4. Exemplary ingredient list in descending order after content in a final pharmaceutical composition according to the current invention, supplier of the ingredient, purity grade and weight percentage.*

| Ingredient list | wt.% Content |
| --- | --- |
| Water | 97-98% |
| Sodium Hyaluronic Acid IV (m3/kg) =2.1 | 2.0% |
| NaOH | 0.11% |
| NaCl | 0.3% |
| Na$_2$HPO$_4$, 12H$_2$O | 0.36% |
| NaH$_2$PO$_4$, 2H2O | 0.08% |
| Peptide (1:1 mix P2&P6) | 2 $\mu$g/mL |

The pharmaceutical composition according to the current invention is based on a hyaluronic acid gel, meaning that the two main ingredients are water and hyaluronic acid. It can also include sodium fluoride and the artificial peptide, that both form a protective mineral layer on the teeth and protect the gum. It also includes citric acid for a fresh taste. Sodium hydroxide is added to modify the pH into the range 6.0-7.0, and sodium chloride, disodium phosphate, and monosodium phosphate are added to give osmolarity.

It is to be understood that while the present invention has been described in conjunction with the detailed description thereof, the foregoing description is intended to illustrate and not limit the scope of the invention, which is defined by the scope of the appended claims.

Other aspects, advantages, and modifications are within the scope of the following claims.

The present invention is further illustrated by the following non-limiting experiments.

EXAMPLES

The following Examples have been included to provide guidance to one of ordinary skill in the art for practicing representative embodiments of the presently disclosed subject matter. Considering the present invention and the general level of skill in the art, those of skill can appreciate that the following Examples are intended to be exemplary only and that numerous changes, modifications, and alterations can be employed without departing from the scope of the presently disclosed subject matter.

**Example 1**

The aim of this example was to compare two crosslinked hyaluronic acid gels prepared using two different crosslinking agents, BDDE and PEGDE. For both agents the crosslinking can be induced through a similar process, thus the aim was to understand if the two agents gave different physio-chemical properties by means of rheology, FTIR, NMR and SEM. One aim was also to understand the nature of the process steps, which was done through *in situ* monitoring of the crosslinking kinetics with rheology, and the use of a design of experiment scheme to understand how the variables hyaluronic acid concentration, time and temperature affected the rheological properties.

**Materials & Method**

Materials

Pharmaceutical grade high molecular weight (Mw = 1.5MDa, IV = 22.2 $m^3$/kg) hyaluronic acid was kindly supplied by Fidia Farmaceutici S.p.A. (Abano Terme, Italy). 1,4-butanediol diglycidyl ether (BDDE), poly(ethylene glycol) diglycidyl ether (PEGDE, $M_n$ = 500 Da), anhydrous sodium hydroxide and sodium chloride was supplied from Merck KGaA, Germany. Potassium bromine (99%, IR grade) was purchased from J&K Scientific Gmbh, Germany.

**Gel production**

Hyaluronic acid was dissolved at 10 w/v% in 0.3M NaOH solution under manual agitation. 1.6 v/v% BDDE or 3.0 v/v% PEGDE was added and stirred in. The solution was incubated for 4 hours at 40 °C in a closed container. Thereafter, the gel was transferred to a cellulose membrane (MWC = 14 kDa) and dialysed in distilled water for 18 hours. The gel was granulated by extruding it through a 130 $\mu$m pored mesh before the final concentration of 20 mg/mL was obtained by further addition of distilled water.

**Rheology**

Rheology of gel samples

The viscoelastic region of the gel samples was investigated using amplitude sweeps by applying a shear strain logarithmic ramp between 0.01-100 % at a 10 rad/s frequency. Further, frequency sweep analysis was applied to understand the crosslinking properties of the gel across a frequency range of 0.1-100 rad/s at a 0.1% shear strain.

**Real-time crosslinking analysis**

The kinetics of the gel crosslinking was conducted using rheology. The dissolved hyaluronic acid was mixed with the crosslinked and immediately transferred to the rheology machine where the measurement was started. Over a 5-hour-time period under constant shear strain of 0.1% and 10 rad/s frequency, the crosslinking kinetics at 40 °C were observed by monitoring the change in viscoelastic properties.

**Material characterisation**

*FTIR*

A FTIR spectrometer (Varian 640-IR, Agilent Technologies, Santa Clara, CA, ISA) was employed to determine the infrared spectra of pure HA, BDDE, PEGDE, and synthesized HA-BDDE and HA-PEGDE hydrogels. Spectra were recorded using KBr pellets in the 400-4000 $cm^{-1}$ range at a 4 $cm^{-1}$ resolution and 50 scans/spectrum. For the gel samples 300 mg KBr and 20 mg of gel was used.

*Morphological characterization*

After the crosslinking step, samples were dehydrated in baths of increasing concentration of ethanol up to absolute ethanol, then dried overnight at 60 °C and 10% humidity. Environmental scanning electron microscopy analysis was performed on gold sputtered samples at 15 kV with Evo 50 EP Instrumentation (Zeiss, Jena, Germany)

*NMR*

**Liquid NMR**

NMR spectroscopy was used to investigate the hyaluronic acid functionalization, and the degree of crosslinking of the gels and to confirm the removal of unreacted crosslinking agent after dialysis.

$^1$H-NMR spectra of the lyophilized gels diluted in deuterated water (4 mg/mL) were collected on a Bruker Advance NMR spectrometer operating at 400 MHz and 298 K. The deuterated solvent (D2O) was used as an internal deuterium lock. Chemical shifts ($\delta$) are reported in parts per million (ppm) using the residual D2O as a reference. Spectra were processed and visualized with MestReNova x64 (Mestrelab Research).

Integrations of the signals at 1.60 and 2.0 ppm ($I^{\delta H1.60}$ and $I^{\delta H2.0}$) were employed to measure the degree of modification (MoD), i.e., the moles of crosslinker bound per disaccharide unit (usually expressed as a percentage), of HA-BDDE gels:

$$MoD = \frac{\left(I^{\delta H1.60}/4\right)}{\left(I^{\delta H2.0}/3\right)} * 100 \qquad (0.1)$$

For HA-PEGDE hydrogel the peaks with a chemical shift in the range 3.20-4.0 ppm include both the HA protons and the PEGDE protons due to the overlap of the signals. Although PEGDE protons can be assigned to the peak at 3.68 ppm, cannot be integrated separately so the integral of the region 3.20-4.0 ppm ($I^{\delta H3.20-4.0}$) must be subtracted by the 10 protons of HA in this range. The subtracted integral ($I^{\delta H3.20-4.0}_{corrected}$) is then the integral of the PEGDE and 40.52 protons on average are present on the PEGDE residue. The degree of modification can consequently be calculated considering the subtracted integral and the integration of the signals at 2.20 ppm:

$$MoD = \frac{\left(I^{\delta H3.20-4.0}_{corrected}/40.52\right)}{\left(I^{\delta H2.20}/3\right)} * 100 \qquad (0.2)$$

This value is an average degree of modification as crosslinked PEGDE shows a distribution of molar masses.

**Leaching study**

A leaching study was employed to investigate the removal of unreacted crosslinking agent. The gel was produced as described above, and before and after the dialysis, the gel was removed, the concentration of hyaluronic acid was modified to 20 mg/mL, and the sample was granulated as described above. Thereafter 2 g of each sample was moved to a cellulose membrane (MWC = 14 kDa), transferred to an enclosed bottle with 50 mL dH$_2$O. After 7 days 1.8 mL solution was removed from each sample, transferred to an NMR tube, before the liquid phase was removed. The residuals were then dissolved in 1.8 mL deuterated water and analysed with NMR as described above.

**Cytotoxicity testing**

Cytotoxicity testing was performed to understand if any leachable could have a detrimental effect on cell proliferation. The gel was prepared similar to as described above but using sterile saline water instead of distilled water. The samples were transferred to syringes before they were sterilized by autoclaving at 121 °C for 15 minutes. Osteoblast cells (MC3T3-E1) were seeded in a 24-well plate at a concentration of 40 000 cells per well and 1 mL cell medium. Approximately 0.1 mL of the gels were added to inserts (0.4 $\mu$m PET-membrane, Merck) which was then added to the wells. There were used 6 wells for each group, including a positive group (cells killed with Triton X-100 1h before finishing) and a negative control (cells only). The quantitative cytotoxicity evaluation was conducted utilizing LDH activity of the gels, and a CCK8 assay was used to calculate cell viability. The LDH assay detects the amount of LDH that leaks out through the plasma membrane of damaged cells, as a marker of cytotoxicity. A cytotoxicity level of below 30% and cell viability above 70% is counted as acceptable according to the ISO 10993-5.

## Results

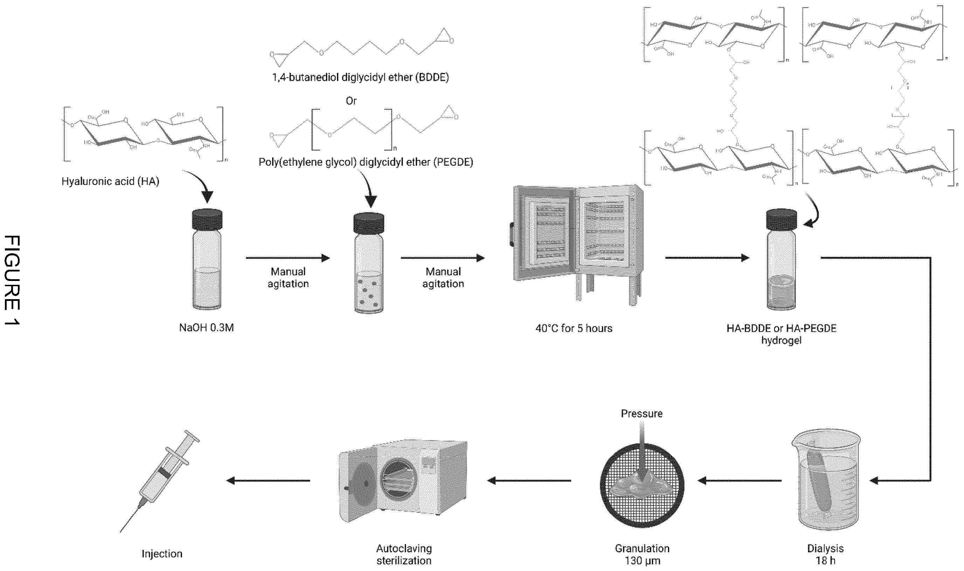

Employing the above-described method, crosslinked hyaluronic acid gels were produced, using both BDDE (1.6 vol.%) and PEGDE (3 vol.%) as crosslinker. The schematic of this process can be found in Figure 1. These two different gels then underwent physio-chemical characterization and cytotoxicity testing. Although a different molar equivalence of the two crosslinkers (BDDE and PEGDE) and the hyaluronic acid was used, the different gels still exhibit similar rheological data, suggesting that the crosslinker plays an essential role in the gels' properties.

*Rheology analysis*

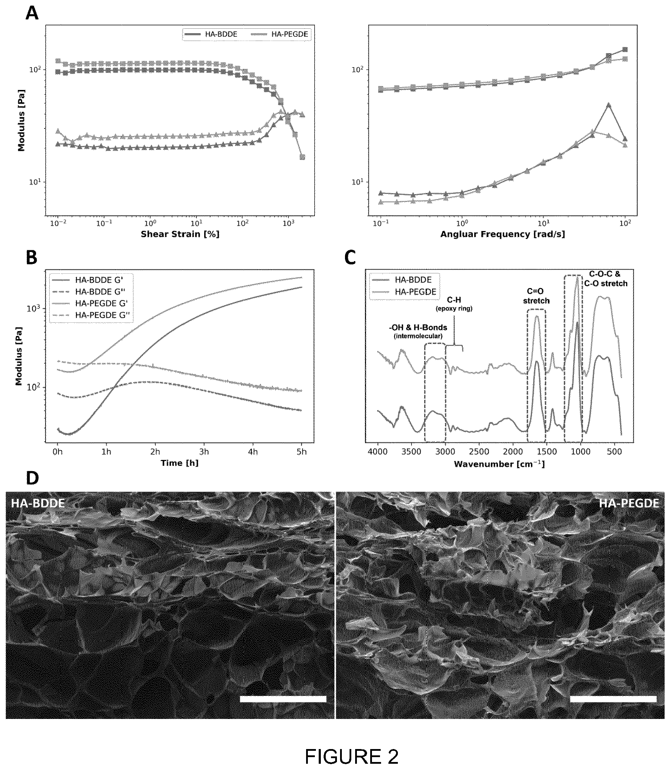

Rheology was applied to understand the mechanical properties of the gel. The gels displayed very similar properties with a wide viscoelastic region reaching up to over a 1000% shear strain (Figure 2A, left panel). Moreover, their frequency sweep behaviour was similar (Figure 2A, right panel). The concentration of PEGDE (3.0 vol.%) was chosen such that the behaviour of the gel mimics that of the 1.6 vol.% BDDE. The rheology results presented in Figure 2 do confirm this. Accordingly, although the molar equivalence between HA and the crosslinker is lower for the PEGDE gel than the BDDE gel the results suggests that the PEGDE crosslinked gel is more elastic than BDDE crosslinked gel.

Further, a real time rheology measure of the *in situ* crosslinking process of the gels was performed **(Figure 2B).** It could be observed that both gel types follow a similar kinetic behaviour, with HA-PEGDE having a quicker kinetic behaviour than HA-BDDE, reaching the gelation point (G' = G") after approximately 30 minutes, where the HA-BDDE gel reaches gelation after roughly 1 hour. They do seem to converge towards a G' of around 1 MPa for HA-BDDE and 2 MPa for HA-PEGDE, which indicates a higher structural stability for HA-PEGDE than for HA-BDDE, in line with the shear strength results presented above.

*Chemical characterisation*

To characterize the material properties from a chemical perspective, FTIR and NMR was applied. From the FTIR analysis, multiple peaks expected from crosslinked hyaluronic acid were detected (Figure 2C). In Figure 2C the peak at 1650 cm$^{-1}$ is the carboxylate group (C=O) of the hyaluronic acid, meanwhile, the wide double peaks at 1050-1150 cm$^{-1}$ are the C-O-C & C-O stretch typical of the ether bonds that are formed during the crosslinking. This suggests that a crosslinked hyaluronic acid gel is achieved both with BDDE and with PEGDE as the crosslinker. Minimal traces of peaks at 2900-3000 cm$^{-1}$, are seen, which are associated with the epoxy end-groups of the crosslinkers that binds to the hydroxyl groups of the HA, which suggests that non-reacted BDDE or PEGDE were successfully removed during the dialysis step. Lastly, there is a wide peak between 3050-3300 cm$^{-1}$ which is associated with intermolecular -OH groups and H-bonds. These intermolecular bonds are essential for the coherences of the gel after granulation and gives them their "sticky" properties which can be clinically favourable.

*Morphological characterization*

After crosslinking step samples were dehydrated in baths of increasing concentration of ethanol up to absolute ethanol, then dried overnight at 60 °C and 10% humidity. Environmental scanning electron microscopy analysis was performed on gold sputtered samples at 15 kV with Evo 50 EP Instrumentation (Zeiss, Jena, Germany).

*Leaching study*

A leaching study was employed to investigate the removal of unreacted crosslinking agent. The gel was produced as described above, and before and after the dialysis gel was removed, the concentration of hyaluronic acid was modified to 20 mg/mL, and the sample was granulated as described above. Thereafter 2 g of each sample was moved to a cellulose membrane (MWC = 14 kDa), transferred to an enclosed bottle with 50 mL pureH2O. After 7 days 1.8 mL solution was removed from each sample, transferred to an NMR tube, before the liquid phase was removed. The residuals were then dissolved in 1.8 mL deuterated water and analysed with 1H NMR as described above.

**Discussion**

The inventors have characterized two different crosslinked hyaluronic acid hydrogels, crosslinked using either BDDE or PEGDE as the crosslinker. The crosslinking can be induced using a similar methodology in both cases, and also, a granulation step was conducted, such that it is compatible with needle injection in a clinical setting. The process starts with the dissolving of hyaluronic acid. Since it typically has a solubility limit of about 4 mg/mL, dependent on salt and molecular weight, it is necessary to dissolve it in a basic/alkaline environment that was ensured using sodium hydroxide (NaOH). The basic/alkaline environment partially deprotonates the hydroxyl groups of the HA molecules, which increases their reactivity, thus facilitating the reaction with the epoxide groups of BDDE to enable the cross-linking. As no or very little cross linking was achieved at HA concentration of 5 wt.% or lower, a concentration of 10 wt.% HA was used. Once the hyaluronic acid was dissolved, the crosslinkers could be readily admixed. The crosslinking process is a time dependent process, that may be accelerated with elevated temperatures. This is beneficial as the high pH accelerates the degradation of hyaluronic acid, resulting in competing reactions between the BDDE or PEGDE crosslinking and HA degradation. In the real-time crosslinking study, a gelation point after approximately 30 minutes or 60 minutes could be observed for the PEGDE or BDDE gel, respectively, when incubated at 40 °C. Both gels seemed to follow a sigmoidal crosslinking kinetic, where the increase in crosslinking decreased with time.

From an industrial perspective it can be beneficial to limit the crosslinking time out of economic considerations. In the experimental setting, crosslinking was stopped after 4 hours. This can be done by neutralizing the pH, with an acid such as e.g., HCl, but it may also be done by transferring the gel into a dialysis solution. The dialysis step is intended to allow the non-reacted crosslinking agent to diffuse out of the gel.

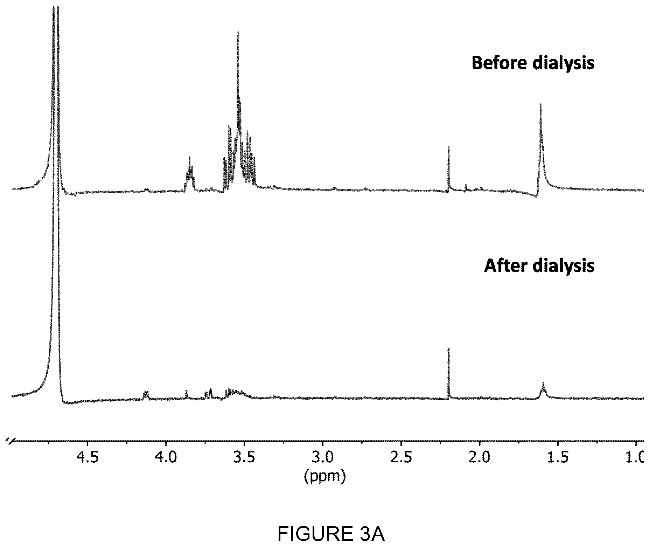

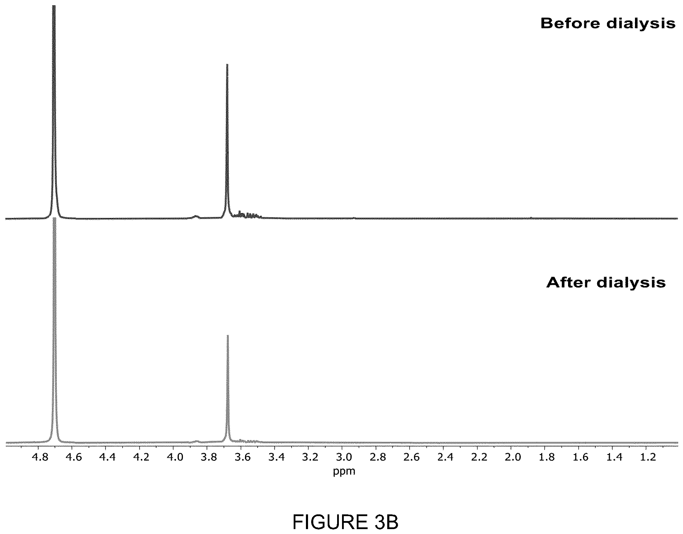

To understand the efficacy of the dialysis step, a leaching study was performed on the gel before and after dialysis, using NMR to characterize the leached composition. Before the dialysis, the peaks associated with the crosslinkers are found in the range from 3.3-3.9ppm for BDDE, and 3.5-3.75 ppm for PEGDE (Figure 3), whereas after 18 hours of dialysis, the peaks in these regions have faded, indicating a that diffusion effectively removes unreacted BDDE and PEGDE from the gels. Further removal of unreacted crosslinking agent may be obtained by e.g., increasing the volume of the dialysis solution, or by exchange of the dialysis medium. The latter was implemented by changing the dialysis medium after two hours, before dialyzing for another 16 hours. Diffusion is also time dependent, so increasing the time can also assist on the removal. For *in vivo* applications it is desired that the dialysis is conducted into a solution suitable for *in vivo* use in order to obtain the adequate osmolarity, such solutions are e.g., PBS buffer or saline water.

**Example 2**

**Production of formulation consisting of hyaluronic acid crosslinked with BDDE and consensus peptide**

1. Hyaluronic acid (3.1 m$^3$/kg) was dissolved in 0.3M NaOH at a concentration of 100 mg/mL under agitation.
2. 16 $\mu$L/mL 1,4-butanediol diglycidyl ether (BDDE) was admixed to the HA solution.
3. The solution was incubated for 4h in a closed container at 40 °C to allow crosslinking,
4. The gel was neutralized using 1M HCl and shaked mildly overnight.
5. The gel was transferred to a cellulose membrane with a cut-off molecular weight of 14 kDa, before it was dialyzed in sterile phosphate-buffered saline (PBS) for 18 hours.
6. The gel was granulated by extruding it through a stainless-steel mesh with pore size of 200 $\mu$m.
7. Separately a solution of phosphate-buffered saline, hyaluronic acid, sodium fluoride, and consensus peptide was created as following:

   a. Hyaluronic acid equivalent to 10 wt.% of the final solution was added to PBS and hydrated by stirring over 1h.
   b. In parallel, the peptide (P2 or P6) solution was created by dissolving the peptide in in sterile water at a concentration of 10 mg/mL.
   c. The peptide solution was added to the PBS-hyaluronic acid solution for a final mixture concentration between 1-500 $\mu$g/mL gel.

8. The PBS-hyaluronic acid solution was added to the granulated crosslinked hyaluronic acid-BDDE and mixed in. The solution was allowed to homogenize under agitation for 6 hours.
9. The gel was transferred to an appropriate delivery system and sterilized using autoclaving.

## Example 3

**Production of formulation consisting of hyaluronic acid crosslinked with BDDE and peptide, optionally doped with 2000 ppm NaF**

The hyaluronic acid gel crosslinked with BDDE was prepared in a similar manner as in example 2. Afterwards, the solution of PBS, hyaluronic acid, and peptide was produced as in example 2 with the small change that NaF is doped into a final concentration of 2000 ppm. The gel and the solutions were mixed together, transferred to a delivery system and sterilized using autoclaving.

## Example 4

**Formulation comprising non-crosslinked hyaluronic acid, a peptide, and sodium fluoride**

1. 50 mg/mL hyaluronic acid was dissolved in 0.3M NaOH using manual stirring.
2. The concentration is modified with sterile PBS such that the concentration of hyaluronic acid at the end of the process was 20 mg/mL.
3. 100 ng/mL to 25 $\mu$g/mL of consensus peptide was added and mixed in.
4. The solution is neutralized with the addition of 1 M HCl and stirred for homogenization,
5. 2000 PPM NaF is added and mixed in.

## Example 5

**Release of biotin-labelled peptide from hyaluronic acid solution and hyaluronic acid gel**

The gels from examples 2-4 were produced with 500 $\mu$g/mL of Biotin labelled P6-peptide. 0.2 mL of the gel was added to 24-well inserts (0.4 $\mu$m, PET), and 1 mL of distilled water was added to each well. After 3h, 7h, and 12h, 1$\mu$L of the water was extracted and the light absorption at 205 nm was read off using a NanoDrop One (Thermo Scientific, US), and compared to a reference curve of 1, 10, 25, and 100 $\mu$g/mL P6-Biotin in distilled water. The reference curve was used to convert to a mass release and a %-release curve was plotted demonstrating that after 12h the un-crosslinked gel (example 2) has released 91% of its peptide load, meantime the crosslinked gel (example 1) has released 23%. This demonstrates that the different way to prepare the gel may be used to tube the release of the peptide and shows that a combination of crosslinked and non-crosslinked gel can give a more controlled and tuneable peptide release compared to non-cross-linked gel only.

## Example 6

**Intra-articular injection in animals with meniscectomy**

The purpose of this example is to demonstrate that the gel solution can lubricate the joint and prevents the progression of osteoarthritis.

*Materials and method:* The gel from example 1 is used but without sodium fluoride and citric acid.

*Procedure:* Twelve adult (4- to 5-yrs old) Ovella Galega sheep were subjected to bilateral lateral meniscectomy. Immediately and up until scarification (12 weeks) the animals were subject to biweekly intra-articular injection of 2 ml of either saline water (control) or hyaluronic acid loaded with the P6 peptide.

*Expected results:* The injection of the P6-loaded hyaluronic acid is expected to reduce the progress of osteoarthritis compared to saline injection alone.

## Example 7

**Intra-articular injection**

The purpose of this example is to demonstrate that the gel solution can lubricate the joint and thereby give pain relief from osteoarthritis.

*Materials and method:* The gel from example 1 is used but without sodium fluoride and citric acid.

*Procedure:* The treatment will be applied to a patient group demonstrating symptoms of mild to moderate osteoarthritis in the knee. The synovial fluid of the join is first removed by extraction through a cannula. Thereafter 3 mL of HA-gel is injected into the joint replacing the synovial fluid.

*Expected results:* The treatment would be expected to reduce pain related to osteoarthritis for at least 26 weeks, with the peptide loaded gel giving improved pain relief compared to the hyaluronic acid alone.

### Example 8

**Bacteriostatic effect of the gel on *Staphylococcus aureus***

This example describes the gel's ability to reduce the bacterial colonization.

*Method:*

*Staphylococcus aureus* is seeded into a 24-well plate allowed to incubation for 24 hours. After this 0.1 mL of: 1) hyaluronic acid solution (20 mg/mL), 2) hyaluronic acid with 1 $\mu$g/mL P2 and 1 $\mu$g/mL P6, and 3) hyaluronic acid with 1 $\mu$g/mL P2 and 1 $\mu$g/mL P6 (from example 10) , or optionally with 0.2% NaF (gel from example 3), is added to the wells (n=6 of each). 6 wells are kept as a negative control with only bacteria. The bacteria is incubated for 48 hours, after which the bacteria load is counted.

*Expected results:*

The hyaluronic acid by itself is expected to stimulate the bacterial colonization compared to the negative control. The hyaluronic acid with the peptides are expected to inhibit bacterial growth, meanwhile the bacteriostatic effect is even more profound with sodium fluoride included.

### Example 9

**Production of formulation consisting of hyaluronic acid crosslinked with BDDE and consensus peptide**

1. Hyaluronic acid (3.1 m$^3$/kg) was dissolved in 0.3M NaOH at a concentration of 100 mg/mL under agitation.
2. 16 $\mu$L/mL 1,4-butanediol diglycidyl ether (BDDE) was admixed to the HA solution.
3. The solution was incubated for 4h in a closed container at 40 °C to allow crosslinking,
4. The gel was neutralized using 1M HCl and shaked mildly overnight.
5. The gel was transferred to a cellulose membrane with a cut-off molecular weight of 14 kDa, before it was dialyzed in sterile phosphate-buffered saline (PBS) for 18 hours.
6. The gel was granulated by extruding it through a stainless-steel mesh with pore size of 200 $\mu$m.
7. Separately a solution of phosphate-buffered saline, hyaluronic acid, and consensus peptide was created as following:

   a. Hyaluronic acid equivalent to 10 wt.% of the final solution was added to PBS and hydrated by stirring over 1h.
   b. In parallel, the peptide (P2 or P6) solution was created by dissolving the peptide in in sterile water at a concentration of 10 mg/mL.
   c. The peptide solution was added to the PBS-hyaluronic acid solution for a final mixture concentration between 1-500 $\mu$g/mL gel.

8. The PBS-hyaluronic acid solution was added to the granulated crosslinked hyaluronic acid-BDDE and mixed in. The solution was allowed to homogenize under agitation for 6 hours.
9. The gel was transferred to an appropriate delivery system and sterilized using autoclaving.

### Example 10

**Production of formulation consisting of hyaluronic acid crosslinked with BDDE and peptide**

The hyaluronic acid gel crosslinked with BDDE was prepared in a similar manner as in example 9. Afterwards, the solution of PBS, hyaluronic acid, and peptide was produced as in example 9. The gel and the solutions were mixed together, transferred to a delivery system and sterilized using autoclaving.

**Example 11**

**Formulation comprising non-crosslinked hyaluronic acid, a peptide, and sodium fluoride**

1. 50 mg/mL hyaluronic acid was dissolved in 0.3M NaOH using manual stirring.
2. The concentration is modified with sterile PBS such that the concentration of hyaluronic acid at the end of the process was 20 mg/mL.
3. 100 ng/mL to 25 µg/mL of consensus peptide was added and mixed in.
4. The solution is neutralized with the addition of 1 M HCl and stirred for homogenization.

**Claims**

1. A pharmaceutical formulation in the form of a gel comprising

   a. an artificial peptide comprising, or consisting of, the amino acid sequence: Pro-X-X-Pro-Y-Y-Y-Pro-X-X-Pro-Y-Y-Pro-X-X-Pro-X-Pro-Y-Y-Y-Y-Y-Y-Pro-Y-Y-Y-Y-Y-Y-Pro-X-X-Pro-X-Pro-Y-Y-Y-Pro-Y-Y-Pro-Y-Pro-X-X-Pro-Y-Pro-Y-Y-Pro-X-X-Pro-Y-Y-Pro-X-X-Pro-Y-Y-Pro-X-X-Pro-Y-Pro-Pro-X-Pro-Pro-X-X-X-X-X-X-X-Pro-X-X-Pro-X-X-X-X (SEQ ID NO 1), wherein

      i) Pro is proline;
      ii) X is an amino acid selected from the group consisting of Ala, Ile, Leu, Met, Phe, Trp and Val;
      iii) Y is an amino acid selected from the group consisting of Asn, Cys, Gln, Ser, Thr and Tyr,

   b. 1-40mg/mL Hyaluronic fibres (HA) and/or 1-40 mg/mL cross-linked Hyaluronic fibres (HA-XL),

   for use in treating an inflamed, degenerated and/or damaged joint.

2. A pharmaceutical formulation in the form of a gel comprising

   a. an artificial peptide comprising, or consisting of, the amino acid sequence: Pro-X-X-Pro-Y-Y-Pro-X-X-Pro-Y-Y-Pro-X-X-Pro-Y-Y-Pro-Y-Pro-Pro-X-Pro-Pro (SEQ ID NO 2), wherein

      i) Pro is proline;
      ii) X is an amino acid selected from the group consisting of Ala, Ile, Leu, Met, Phe, Trp and Val; and
      iii) Y is an amino acid selected from the group consisting of Asn, Cys, Gln, Ser, Thr and Tyr,

   b. 1-40mg/mL Hyaluronic fibres (HA) and/or 1-40 mg/mL cross-linked Hyaluronic fibres (HA-XL),

   for use in treating an inflamed, degenerated and/or damaged joint.

3. A pharmaceutical formulation for use according to claim 1 or 2, wherein the artificial peptide is at least 90% identical to an artificial peptide selected from the group consisting of the amino acid sequences of SEQ ID NO 1, SEQ ID NO 2, SEQ ID NO 3, SEQ ID NO 4, and SEQ ID NO 5, such as wherein the artificial peptide is one or more artificial peptide(s) selected from the group consisting of artificial peptides comprising the amino acid sequence of SEQ ID NO 4 and SEQ ID NO 5.

4. A pharmaceutical formulation for use according to any of the preceding claims, comprising

   a. 0.1-250 µg/mL, such as 0.1, 1.0, 5.0, 10, 50, 100, 200 or 250 µg/mL of said artificial peptide,
   b. 1-40 mg/mL, such as 1.0, 2.5, 4, 10, 20, 25, 30 or 40 mg/mL cross-linked Hyaluronic fibres (HA-XL), and/or
   c. 1-40 mg/mL, such as 1.0, 2.5, 4, 10, 20, 25, 30 or 40 mg/mL non-crosslinked Hyaluronic fibres (HA).

5. A pharmaceutical formulation for use according to any one of the preceding claims, wherein the cross-linked Hyaluronic fibres (HA-XL) comprise or consist of 1,4-butanediol diglycidyl ether cross-linked Hyaluronic fibres (HA-BDDE) and/or poly(ethylene glycol) diglycidyl ether (PEGDE) cross-linked Hyaluronic fibres (HA-PEGDE).

6. A pharmaceutical formulation for use according to any of the preceding claims comprising

   a. 50 μg/mL of an artificial peptide with the amino acid sequence of SEQ ID NO 4 and/or SEQ ID NO 5,
   b. 20 mg/mL 1,4-butanediol diglycidyl ether cross-linked Hyaluronic fibres (HA-BDDE) and/or poly(ethylene glycol) diglycidyl ether cross-linked Hyaluronic fibres (HA-PEGDE), and
   c. 2.5 mg/mL non-crosslinked Hyaluronic fibres (HA).

7. A pharmaceutical formulation for use according to any of the preceding claims comprising

   a. 2 μg/mL of an artificial peptide with the amino acid sequence of SEQ ID NO 4 and/or SEQ ID NO 5,
   b. 20 mg/mL 1,4-butanediol diglycidyl ether cross-linked Hyaluronic fibres (HA-BDDE) and/or poly(ethylene glycol) diglycidyl ether cross-linked Hyaluronic fibres (HA-PEGDE), and
   c. 2.5 mg/mL non-crosslinked Hyaluronic fibres (HA).

8. A pharmaceutical formulation for use according to any of the preceding claims, wherein the Hyaluronic acid fibres are between 0.7-4.0 MDa, such as between 1.0-2.0, between 1.5-1.8, or between 3.0-3.3 MDa, such as 1.5MDa.

9. A pharmaceutical formulation for use according to claim 6, 7 or 8, further comprising

   d. one or more buffering agent(s)
   e. one or more salt(s), and
   f. water.

10. A pharmaceutical formulation for use according to claim 6, 7 or 8, further comprising

   d. Water,
   e. Sodium Hydroxide,
   f. Sodium Chloride,
   g. Disodium Phosphate and
   h. Sodium Phosphate.

11. A pharmaceutical formulation for use according to any of the preceding claims, wherein the artificial peptide is released at a controlled rate of 0.01-10 ug pr hour, such as about 0.01, 0.1, 1, 2, 3, 4, 5, 6, 7, 8, 9 or 10 ug per hour.

12. A pharmaceutical formulation for use according to any of the preceding claims, wherein the pharmaceutical formulation induces and/or supports tissue healing and/or regeneration in a joint and/or in tissue adjacent to a joint.

13. A pharmaceutical formulation for use according to any of the preceding claims, wherein the pharmaceutical formulation reduces and/or suppresses an infection and/or inflammation in a joint and/or in tissue adjacent to a joint.

14. A pharmaceutical formulation for use according to any of the preceding claims, wherein the pharmaceutical formulation stimulates collagen production in joint tissue and/or promotes oriented collagen formation in joint tissue.

15. A pharmaceutical formulation for use according to any of the preceding claims, for use in preventing, ameliorating, preventing progression of and/or treating arthritis.

16. A pharmaceutical formulation for use according to any of the preceding claims, for use in viscosupplementation.

17. Administration of a pharmaceutical formulation for use according to any of the preceding claims, wherein the pharmaceutical formulation is administered as a minimally invasive administration by injection.

FIGURE 1

FIGURE 2

FIGURE 3A

FIGURE 3B

FIGURE 4

Europäisches
Patentamt

European
Patent Office

Office européen
des brevets

# EUROPEAN SEARCH REPORT

**Application Number**

EP 23 18 4221

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2022/176020 A1 (PERALE GIUSEPPE [CH] ET AL) 9 June 2022 (2022-06-09)<br>* paragraph [0187] *<br>* paragraph [0094] – paragraph [0117]; claims 1,13, 25; examples 2B, 2C, 2D * | 1-17 | INV.<br>A61K38/08<br>A61K31/728<br>A61K38/10<br>A61L27/52<br>A61P19/02 |
| Y | WO 2008/078167 A2 (CORTICALIS AS [NO]; LYNGSTADAAS STALE PETTER [NO] ET AL.) 3 July 2008 (2008-07-03)<br>* page 4, lines 25-28 *<br>* page 24, lines 21-25; claims 10,29-32,58; examples 1,5 * | 1-17 | |
| Y | WO 2016/002983 A1 (MEDYTOX INC [KR]) 7 January 2016 (2016-01-07)<br>* paragraphs [0021], [0025], [0033], [0034]; claims; example 3 * | 1-17 | |
| Y | EP 2 742 070 B1 (GLYCORES 2000 SRL [IT]) 15 September 2021 (2021-09-15)<br>* paragraphs [0002], [0025] – [0026]; claims 1,7,9; example 14 * | 1-17 | |
| A | RUBERT M ET AL: "Effect of TiO 2 scaffolds coated with alginate hydrogel containing a proline-rich peptide on osteoblast growth and differentiation in vitro",<br>JOURNAL OF BIOMEDICAL MATERIALS RESEARCH PART A, JOHN WILEY & SONS, US, [Online] vol. 101a, no. 6, 1 June 2013 (2013-06-01) , pages 1768-1777, XP002716225,<br>ISSN: 1549-3296, DOI: 10.1002/JBM.A.34458<br>Retrieved from the Internet:<br>URL:http://onlinelibrary.wiley.com/doi/10. 1002/jbm.a.34458/pdf><br>* page 1769, left-hand column, paragraph 3-4 *<br>* page 1775, right-hand column * | 1-15 | TECHNICAL FIELDS SEARCHED (IPC)<br>A61K<br>A61L<br>A61P |

The present search report has been drawn up for all claims

| Place of search | Date of completion of the search | Examiner |
|---|---|---|
| Munich | 28 November 2023 | Escolar Blasco, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 4221

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2023

| Patent document cited in search report | | Publication date | Patent family member(s) | | Publication date |
|---|---|---|---|---|---|
| US 2022176020 | A1 | 09-06-2022 | CN | 113543817 A | 22-10-2021 |
| | | | EP | 3714909 A1 | 30-09-2020 |
| | | | EP | 3946482 A1 | 09-02-2022 |
| | | | JP | 2022525813 A | 19-05-2022 |
| | | | KR | 20210145130 A | 01-12-2021 |
| | | | SG | 11202108582S A | 29-09-2021 |
| | | | US | 2022176020 A1 | 09-06-2022 |
| | | | WO | 2020193285 A1 | 01-10-2020 |
| WO 2008078167 | A2 | 03-07-2008 | AT | E475670 T1 | 15-08-2010 |
| | | | AU | 2007337797 A1 | 03-07-2008 |
| | | | CA | 2673382 A1 | 03-07-2008 |
| | | | DK | 2118136 T3 | 15-11-2010 |
| | | | EP | 2118136 A2 | 18-11-2009 |
| | | | ES | 2349889 T3 | 12-01-2011 |
| | | | HK | 1137186 A1 | 23-07-2010 |
| | | | JP | 5253412 B2 | 31-07-2013 |
| | | | JP | 2010513460 A | 30-04-2010 |
| | | | KR | 20090117702 A | 12-11-2009 |
| | | | KR | 20150014999 A | 09-02-2015 |
| | | | PL | 2118136 T3 | 29-04-2011 |
| | | | US | 2010172949 A1 | 08-07-2010 |
| | | | WO | 2008078167 A2 | 03-07-2008 |
| WO 2016002983 | A1 | 07-01-2016 | KR | 20160002256 A | 07-01-2016 |
| | | | WO | 2016002983 A1 | 07-01-2016 |
| EP 2742070 | B1 | 15-09-2021 | CY | 1124718 T1 | 22-07-2022 |
| | | | DK | 2742070 T3 | 08-11-2021 |
| | | | EP | 2742070 A1 | 18-06-2014 |
| | | | ES | 2899992 T3 | 15-03-2022 |
| | | | HR | P20211659 T1 | 04-02-2022 |
| | | | HU | E056131 T2 | 28-01-2022 |
| | | | LT | 2742070 T | 25-10-2021 |
| | | | PL | 2742070 T3 | 31-01-2022 |
| | | | PT | 2742070 T | 22-11-2021 |
| | | | RU | 2014108821 A | 20-09-2015 |
| | | | SI | 2742070 T1 | 30-11-2021 |
| | | | WO | 2013021249 A1 | 14-02-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2118136 A
- WO 03061626 A
- US 4801539 A
- EP 0694616 A
- WO 03054163 A
- WO 9923227 A
- WO 9951265 A
- WO 0027437 A
- WO 9822598 A
- WO 2013030348 A1, Falcone

### Non-patent literature cited in the description

- **BITTER** ; **MUIR**. *Anal Biochem.*, 1962, vol. 4, 330-334
- **DEANGELIS, P. L.** *Cell. Mol. Life Sci*, 1999, vol. 56, 670-682
- *Proc. Natl. Acad. Sci. USA.*, 2001, vol. 98, 4658-4663
- **SVENSSON J** ; **ANDERSSON C** ; **RESELAND JE** ; **LYNGSTADAAS SP** ; **BULOW L.** Histidine tag fusion increase expression levels of active recombinant Amelogenin in Escherichia coli. *Protein Expr Purif*, 2006, vol. 48, 134-41
- Encyclopedia of Pharmaceutical Technology. Marcel Dekker, Inc., 1988
- Remington's Pharmaceutical Sciences. Mack Publishing Company, 1990